(19) Europäisches Patentamt
European Patent Office
Office européen des brevets

(11) **EP 2 657 343 B1**

(12) **EUROPEAN PATENT SPECIFICATION**

(45) Date of publication and mention
of the grant of the patent:
**05.02.2020  Bulletin 2020/06**

(51) Int Cl.:
***C12P 7/56*** (2006.01)          ***C12M 1/12*** (2006.01)
***C12M 1/36*** (2006.01)

(21) Application number: **11850267.3**

(22) Date of filing: **21.12.2011**

(86) International application number:
**PCT/JP2011/079714**

(87) International publication number:
**WO 2012/086720 (28.06.2012 Gazette 2012/26)**

(54) **METHOD FOR PRODUCING CHEMICAL BY CONTINUOUS FERMENTATION**

VERFAHREN ZUR HERSTELLUNG EINER CHEMIKALIE DURCH KONTINUIERLICHE
FERMENTIERUNG

PROCÉDÉ DE PRODUCTION D'UNE SUBSTANCE CHIMIQUE PAR FERMENTATION CONTINUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2010  JP 2010285365
25.02.2011  JP 2011039572**

(43) Date of publication of application:
**30.10.2013  Bulletin 2013/44**

(73) Proprietor: **TORAY INDUSTRIES, INC.**
**Tokyo 103-8666 (JP)**

(72) Inventors:
• **TAKEUCHI, Norihiro**
**Otsu-shi**
**Shiga 520-8558 (JP)**
• **CHEON, Jihoon**
**Otsu-shi**
**Shiga 520-8558 (JP)**
• **NISHIDA, Makoto**
**Otsu-shi**
**Shiga 520-8558 (JP)**
• **KANAMORI, Satoko**
**Otsu-shi**
**Shiga 520-8558 (JP)**

• **KOBAYASHI, Atsushi**
**Otsu-shi**
**Shiga 520-8558 (JP)**

(74) Representative: **Hoefer & Partner Patentanwälte
mbB**
**Pilgersheimer Straße 20**
**81543 München (DE)**

(56) References cited:
**EP-A2- 0 188 068      JP-A- 2 006 824
JP-A- 2008 161 071     JP-A- 2008 161 071
JP-A- 2009 065 966     JP-A- 2010 253 357**

• **DATABASE WPI Week 200929 Thomson
Scientific, London, GB; AN 2009-G56461
XP002764917, -& JP 2009 065966 A (TORAY IND
INC) 2 April 2009 (2009-04-02)**
• **DATABASE WPI Week 200865 Thomson
Scientific, London, GB; AN 2008-L05102
XP002764918, -& JP 2008 161071 A (TORAY IND
INC) 17 July 2008 (2008-07-17)**
• **DATABASE EPODOC [Online] EUROPEAN
PATENT OFFICE; "ALCOHOL SEPARATOR",
XP002764919, Database accession no.
JP-2009104571-A -& JP 2010 253357 A
(SUMITOMO ELECTRIC INDUSTRIES) 11
November 2010 (2010-11-11)**

**Description**

Field

The present invention relates to a method for producing a chemical by continuous fermentation in which a chemical is produced continuously by fermentation using separation membranes.

Background

The fermentation method as a method of producing a substance, which involves culturing a microorganism or culture cells, can be roughly classified into (1) a batch fermentation method and a fed-batch fermentation method, and (2) a continuous fermentation method.

The above (1) batch fermentation method and fed-batch fermentation method have advantages such as simple facilities and less damage by contaminating bacteria because culture is completed in a short time. However, the concentration of a chemical product in a fermentation culture liquid is increased with time to lower productivity and yield due to the influence of osmotic pressure or inhibition by the chemical product. Accordingly, it is difficult to maintain stably high yield and high productivity for a long time.

The above (2) continuous fermentation method is characterized in that high yield and high productivity can be maintained for a long time by preventing accumulation of an objective chemical at high concentration in a fermentation tank. Continuous fermentation methods for fermentation of L-glutamic acid and L-lysine have been disclosed (see, Non-Patent Literature 1). In these examples, however, raw materials are continuously fed to a fermentation culture liquid while a fermentation culture liquid containing microorganisms or culture cells is withdrawn, so that the microorganisms and culture cells in the fermentation culture liquid are diluted, and therefore the improvement in production efficiency is limited.

In the continuous fermentation method, it has been proposed that microorganisms and culture cells are filtered with a separation membrane to recover a chemical product from a filtrate, while the microorganisms and culture cells in a concentrated liquid are retained in, or returned to, a fermentation culture liquid thereby maintaining a high density of the microorganisms and culture cells in the fermentation culture liquid. For example, techniques of continuous fermentation in a continuous fermentation apparatus which uses a flat sheet membrane consisting of organic polymers as a separation membrane have been disclosed (see, Patent Literature 1).

Meanwhile, with respect to separation membrane, it is used for various fields such as water treatment including production of a drink, water purification, and waste water treatment, and field of food industry as well as an application to fermentation field as described above. In the water treatment field including production of a drink, water purification, and waste water treatment, a separation membrane is used for removing impurities in water as a substitute for processes of sand filtration and aggregational precipitation. Since water treatment amount is high in the water treatment field including water purification and waste water treatment, improved permeablity is required. In this regard, by decreasing the membrane area with use of a separation membrane with excellent permeablity and by using a hollow fiber membrane module or a spiral type module which has small installation area per membrane area, it is tried to develop a more compact apparatus and to lower the cost associated with having facilities and membrane exchange.

For more efficient production by continuous fermentation using a continuous fermentation apparatus, a technique of using a hollow fiber module or the like having small installation area per membrane area and low exchange cost for separation membrane module is disclosed (see, Patent Literature 2). According to the technique, it is suggested that, by using a hollow fiber membrane as a separation membrane, microorganisms and culture cells are filtered to recover a chemical from a filtrate, while the microorganisms or culture cells in the concentrated liquid are simultaneously retained in, or returned to, a fermentation culture liquid thereby maintaining a high concentration of the microorganisms and culture cells in the fermentation culture liquid. A cross flow filtration is adopted in which the fermentation culture liquid is transported to a hollow fiber membrane module, part of the liquid is filtered, and most of them are returned to a fermentation tank. According to shear force generated by cross flow motion, fouling on membrane surface are removed so that efficient filtration can be carried out for a long period of time.

Herein, from the viewpoint of having facilities required for industrialization, there could be fermentation using a fermentation tank which is as big as several hundred m$^3$. For filtering fermentation liquid containing microorganisms at high concentration, a large membrane area is needed, and for such reasons, plural separation membrane modules are used. For example, when fermentation liquid of 100 m$^3$ is to be filtered, although the optimum number may vary depending on filtration property of a fermentation liquid and performance of a separation membrane module, plural separation membrane modules that are as many as several hundreds or several thousands are required.

As to an operation method of a separation membrane module, to maintain a period for performing continuous fermentation or good filtration property, a technique of removing precipitates on surface of a separation membrane based on shear force generated by cross flow motion following intermittent filtration (see, Patent Literature 3) or a technique of removing precipitates present inside a separation membrane following backwashing using a pH control liquid during no filtration period (see, Patent Literature 4) is disclosed.

Patent Literature 4 further discloses:
a method for producing a chemical by continuous fermentation comprising:

a fermentation step for converting a fermentation feedstock, through fermentation by culturing a microorganism or culture cells, into a fermented liquid containing the chemical by a fermentation tank, and

a membrane separation step for collecting the chemical, as a filtrate, from the fermented liquid with the use of a plurality of separation membrane modules, and returning the non-filtered liquid in the fermented tank,wherein in the membrane separation step, an intermittent filtration treatment is performed such that a filtration treatment and a filtration-stop treatment are alternately repeated with the plurality of the separation membrane modules and using a plurality of the separation membrane modules that are arranged in parallel, and the timing of the filtration-stop treatment in each separation membrane module is controlled during the intermittent filtration treatment.

Meanwhile, regarding the field of water treatment, a technique of lowering washing liquid amount, waste water amount, and the amount of air used for washing separation membrane is disclosed in which, during filtration, raw liquid is supplied to each module placed in a row to perform filtration, and during washing, each module is connected by flushing pipes, and modules are arranged in series based on operation of opening and closing an opening and closing valve placed at a pre-determined position so that the hollow fiber membrane is subjected to flushing washing is disclosed (Patent Literature 5).

Citation List

Patent Literature

Patent Literature 1: Japanese Laid-open Patent Publication No. 2007-252367
Patent Literature 2: Japanese Laid-open Patent Publication No. 2008-237101
Patent Literature 3: Japanese Laid-open Patent Publication No. 2009-65966
Patent Literature 4: Japanese Laid-open Patent Publication No. 2008-161071
Patent Literature 5: Japanese Laid-open Patent Publication No. 2009-72708

Non Patent Literature

Non Patent Literature 1: Toshihiko Hirao et al., Appl. Microbiol. Biotechnol., 32, 269-273 (1989)

Summary

Technical Problem

However, if the intermittent filtration described in Patent Literature 3 is carried out in such a manner that filtration for nine minutes and filtration-stop for one minutes are repeated to have intermittent filtration, during nine minutes of filtration, feedstock is added to a fermentation tank in the same amount as the fermentation liquid reduced by filtration, but during one minute of filtration-stop, the amount of the fermentation liquid is not reduced so that no feedstock is added to a fermentation tank. Thus, when a continuous fermentation apparatus is used for performing cross flow filtration using plural separation membrane units and the plural separation membrane modules are driven in such a manner that filtration is performed for nine minutes and filtration-stop is performed for one minute, all at the same timing, addition of the feedstock is intermittent, and as a result, the feedstock concentration in a fermentation tank may not be stabilized, making it difficult to achieve stable fermentation.

Further, when the backwashing disclosed in Patent Literature 4 is performed during filtration-stop for the intermittent filtration described above, during nine minutes of filtration, feedstock is added to a fermentation tank in the same amount as the fermentation liquid reduced by filtration, but during one minute of filtration-stop for backwashing, washing liquid from the backwashing is introduced to the fermentation tank, and therefore the amount of fermentation liquid increases in the fermentation tank. For such reasons, until the increased amount of washing liquid from backwashing disappears, no feedstock is added to a fermentation tank. Thus, when plural separation membrane modules are operated to repeat at the same timing the filtration for nine minutes and the filtration-stop and backwashing for one minute, the feedstock is added intermittently, yielding unstable concentration of feedstock in the fermentation tank, and thus it may be difficult to achieve stable fermentation.

In addition, for a case in which water containing either alkali or acid is used as a washing liquid for backwashing, when back liquid washing is performed simultaneously by using plural separation membrane modules, pH of fermentation liquid can fall temporarily outside the optimum range, and as a result, the fermentation performance may be impaired and also activity of microorganisms is lowered during that period.

Further, when filtration of fermentation liquid is performed by using separation modules that are arranged in series as disclosed in Patent Literature 5, total cross flow rate is lowered so that large facilities or cost increase can be prevented. However, when filtration of fermentation liquid is performed by using separation modules that are arranged in series, compared to a front module in serial arrangement, a back module receives lower pressure at primary side as much as the pressure loss caused by liquid flow through the primary side of the front module. As a result, the back module receives smaller transmembrane pressure, and thus there is a problem that the filtration amount is decreased. For fermentation liquid having high microorganism concentration for continuous fermentation, it is necessary to set the cross flow flux at high level. Accordingly, pressure loss of the module is increased when liquid flow is created toward the primary side of separation membrane, and such tendency becomes more significant. In addition, as the filtration amount is larger at the front side module, there is also a problem that membrane clogging is more easily caused than the back side module. In addition, as turbid matter concentration is high in continuous filtration, in order to filter fermentation culture containing microorganisms, it is necessary to prevent precipitation of microorganisms or the like on the surface of primary side of separation membrane based on shear force generated by cross flow.

The present invention is devised in view of the above circumstances, and it relates to, for producing a chemical characterized by filtering and collecting efficiently liquid containing a product after passing through a separation membrane from culture liquid of microorganisms or culture cells while continuously performing culture and obtaining high productivity by increasing concentration of microorganisms involved with fermentation by returning non-filtered liquid to a culture liquid, a method of producing a chemical by which washing of a separation membrane is performed efficiently and also fermentation is performed stably.

Solution to Problem

To solve the problem described above and achieve the object, a method for producing a chemical by continuous fermentation according to the present invention includes: a fermentation step for converting a fermentation feedstock, through fermentation by culturing a microorganism or culture cells, into a fermented liquid containing the chemical by a fermentation tank; and a membrane separation step for collecting the chemical, as a filtrate, from the fermented liquid with the use of a plurality of separation membrane modules, and returning a non-filtered liquid to a fermented tank, wherein in the membrane separation step, an intermittent filtration treatment is performed such that a filtration treatment and a filtration-stop treatment are alternately repeated with the plurality of the separation membrane modules and using the plurality of the separation membrane modules that are arranged in series, and the timing of the filtration-stop treatment in each separation membrane module is controlled during the intermittent filtration treatment.

Moreover, in the method for producing a chemical by continuous fermentation according to the present invention as set forth in the invention described above, the filtration-stop treatment of the separation membrane module is controlled such that stopping the filtering operation of at least one separation membrane module is performed during a filtering operation of other separation membrane module.

Moreover, in the method for producing a chemical by continuous fermentation according to the present invention as set forth in the invention described above, the filtration-stop treatment of the separation membrane module is controlled such that the filtration-stop treatment of each separation membrane module is not overlapped with each other.

Moreover, in the method for producing a chemical by continuous fermentation according to the present invention as set forth in the invention described above, timing of the filtration-stop treatment of the separation membrane module is controlled such that a change in non-filtered liquid amount per hour, which is returned from the separation membrane module to the fermentation tank, can be minimized.

Moreover, in the method for producing a chemical by continuous fermentation according to the present invention as set forth in the invention described above, the membrane separation step is performed by backwashing using water as a washing liquid during the filtration-stop treatment.

Moreover, in the method for producing a chemical by continuous fermentation according to the present invention as set forth in the invention described above, the membrane separation step is performed during the filtration-stop treatment by backwashing using water containing an oxidizing agent or a reducing agent as a washing liquid.

Moreover, in the method for producing a chemical by continuous fermentation according to the present invention as set forth in the invention described above, the membrane separation step is performed during the filtration-stop treatment by backwashing using water containing an acid or an alkali as a washing liquid.

Moreover, in the method for producing a chemical by continuous fermentation according to the present invention as set forth in the invention described above, the membrane separation step is performed during the filtration-stop treatment by immersion washing using a washing liquid.

Moreover, in the method for producing a chemical by continuous fermentation according to the present invention as set forth in the invention described above, timing of the filtration-stop treatment of the separation membrane module is controlled such that an amount of non-filtered liquid which is returned from the separation membrane module to the fermentation tank is almost the same as the amount of washing liquid that is used for backwashing.

Moreover, in the method for producing a chemical by continuous fermentation according to the present invention as set forth in the invention described above, transmembrane pressure is controlled to be constant in each separation membrane module arranged in series.

Moreover, in the method for producing a chemical by continuous fermentation according to the present invention as set forth in the invention described above, order of transporting fermentation liquid to a plurality of the separation membrane modules arranged in series can be varied.

Moreover, in the method for producing a chemical by continuous fermentation according to the present invention as set forth in the invention described above, the membrane separation step comprises performing, in multiple lines in parallel, an intermittent filtration operation using a separation membrane unit which includes a plurality of the separation membrane modules that are arranged in series.

Moreover, in the method for producing a chemical by continuous fermentation according to the present invention as set forth in the invention described above, the membrane separation step comprises performing a filtration treatment by varying pressure of fermentation liquid supplied to primary side of the separation membrane. Advantageous Effects of Invention

According to the invention, washing of a separation membrane can be performed efficiently and also fermentation is performed stably, and thus in fermentation industries, in general, a chemical as a fermentation product can be stably produced at low cost.

Brief Description of Drawings

FIG. 1 is a schematic diagram illustrating a membrane separation type continuous fermentation apparatus, which is operable by arranging in parallel the separation membrane modules.

FIG. 2 is a flowchart for describing intermittent filtration treatment.

FIG. 3 is a flowchart for describing intermittent filtration treatment according to a modification example.

FIG. 4 is a schematic diagram illustrating a membrane separation type continuous fermentation apparatus, which is operated by arranging in series the separation membrane modules according to a first embodiment of the invention.

FIG. 5 is a schematic diagram illustrating a membrane separation type continuous fermentation apparatus, which is operated by arranging in series the separation membrane modules according to a modification example of the first embodiment of the invention.

FIG. 6 is a schematic diagram illustrating a membrane separation type continuous fermentation apparatus, which is operated by arranging in series plural separation membrane modules according to a second embodiment of the invention and arranging in parallel multiple rows of the separation membrane modules which have been arranged in series.

FIG. 7 is a flowchart for describing intermittent filtration treatment according to the second embodiment of the invention.

FIG. 8 is a flowchart for describing intermittent filtration treatment including backwashing performed during filtration-stop treatment according to the second embodiment of the invention.

Description of Embodiments

Herein below, modes for carrying out the invention are described. First, descriptions are given to an outline of the method for producing a chemical by continuous fermentation according to the invention followed by specific embodiments of the invention.

\<Method for producing chemical\>

1. Fermentation step

According to the present embodiment, the method for producing a chemical includes a fermentation step for converting fermentation feedstock into fermentation liquid containing a chemical by fermentation culture of microorganisms.

(A) Microorganisms and culture cells

Herein below, descriptions are made with regard to microorganisms and culture cells.

The microorganisms that can be used for producing a chemical are not particularly limited. Examples of the microorganisms include yeasts such as baker's yeast used frequently in fermentation industry, fungus such as a filamentous fungus, bacteria such as Escherichia coli or coryneform bacteria, and actinobacteria. Examples of the culture cells include animal cells and insect cells. The microorganisms or culture cells used may be those isolated from the natural environment or may be those having properties modified partially by mutation or genetic recombination.

For producing lactic acid, it is preferable to use yeast or lactobacillus for eukaryotic cells and prokaryotic cells, respectively. Of those, the yeast is preferably yeast obtained by introducing genes encoding lactic acid dehydrogenase to cells. Of those, it is preferable to use lactobacillus which produces lactic acid of 50% or more, in terms of yield per sugar, i.e., consumed glucose. It is more preferable to use lactobacillus which produces lactic acid of 80% or more, in terms of yield per sugar.

Examples of the lactobacillus that is preferably used for producing lactic acid include, among wild type strains, bacteria belonging to Lactobacillus, genus Bacillus, Pediococcus, genus Tetragenococcus, genus Carnobacterium, genus Vagococcus, genus Leuconostoc, genus Oenococcus, genus Atopobium, genus Streptococcus, genus Enterococcus, genus Lactococcus, and genus Sporolactobacillus, which have an ability of synthesizing lactic acid.

Further, lactobacillus exhibiting high lactic acid yield per sugar or high optical purity may be selected and used. Examples of the lactobacillus having an ability of producing selectively D-lactic acid include D-lactic acid-producing bacterial belonging to genus Sporolactobacillus. Specific examples of the preferred bacteria that may be used include Sporolactobacillus laevolacticus, and Sporolactobacillus inulinus. More preferred examples include Sporolactobacillus laevolacticus ATCC 23492, ATCC 23493, ATCC 23494, ATCC 23495, ATCC 23496, ATCC 223549, IAM12326, IAM 12327, IAM 12328, IAM 12329, IAM 12330, IAM 12331, IAM 12379, DSM 2315, DSM 6477, DSM 6510, DSM 6511, DSM 6763, DSM 6764, DSM 6771, and Sporolactobacillus inulinus JCM 6014.

Examples of the lactobacillus exhibiting high L-lactic acid yield per sugar include Lactobacillus yamanashiensis, Lactobacillus animalis, Lactobacillus agilis, Lactobacillus aviaries, Lactobacillus casei, Lactobacillus delbruekii, Lactobacillus paracasei, Lactobacillus rhamnosus, Lactobacillus ruminis, Lactobacillus salivarius, Lactobacillus sharpeae, Pediococcus dextrinicus, and Lactococcus lactis, and each of them may be selected and used for producing L-lactic acid.

(B) Fermentation feedstock

Fermentation feedstock can be any materials which promote growth of microorganisms and culture cells to allow satisfactory production of a desired chemical as a fermentation product.

Liquid medium is used as fermentation feedstock. As a component in medium, a material converting into a desired chemical (i.e., feedstock in narrow sense) may be referred to as feedstock. However, in the present document, the entire culture medium is referred to as feedstock, unless specifically described otherwise. Feedstock in narrow sense indicate sugar such as glucose, fructose, sucrose, or the like, which is a substrate for fermentation to obtain alcohols as a chemical.

The feedstock appropriately contains a carbon source, a nitrogen source, inorganic salts, and if necessary, organic micronutrients such as amino acids and vitamins. The carbon source that can be used herein includes sugars such as glucose, sucrose, fructose, galactose and lactose, starch sugars containing these sugars, sweet potato molasses, sugar beet molasses, and high test molasses, organic acids such as acetic acid, alcohols such as ethanol, and glycerin. The nitrogen source that can be used herein includes ammonia gas, ammonia water, ammonium salts, urea, nitrates, and other secondarily used organic nitrogen sources, for example oil cakes, soybean hydrolysates, casein hydrolysates, other amino acids, vitamins, corn steep liquor, yeasts or yeast extract, meat extract, peptides such as peptone, and various fermented microorganisms, and their hydrolysates. The inorganic salts that can be appropriately added include phosphate salts, magnesium salts, calcium salts, iron salts, and manganese salts.

When the microorganisms or culture cells require a specific nutrient for their growth, the nutrient is added to feedstock as a preparation or as a natural product containing the same.

The feedstock may contain an antifoaming agent, if necessary.

(C) Culture medium

The culture medium refers to a liquid obtained as a result of growth of a microorganism or culture cells with the fermentation feedstock.

For continuous fermentation, the fermentation feedstock may be added to culture medium, but composition of added fermentation feedstock may be changed appropriately from the composition at the start of culturing so that productivity of desired chemical can be increased. For example, concentration of fermentation feedstock in narrow sense, concentration of other components in medium, or the like may vary.

(D) Fermentation liquid

Fermentation liquid is a liquid containing materials produced as a result of fermentation, and it may contain feedstock, microorganisms or culture cells, and a chemical. In other words, the expression "culture medium" and "fermentation liquid" may be used interchangeably with the same meaning.

(E) Chemicals

According to the method of the present embodiment, a chemical, i.e., converted material, is produced in a fermentation liquid by the microorganisms or culture cells described above. Examples of the chemical include substances such as alcohols, organic acids, amino acids or nucleic acid that are produced in large amounts in fermentation industry. Examples of alcohols include ethanol, 1,3-propane diol, 1,4-butane diol and glycerol. Examples of organic acids include acetic acid, lactic acid, pyruvic acid, succinic acid, malic acid, itaconic acid, citric acid, and nucleic acids such as inosine, guanosine, and citidine. The method of the invention can also be applied to production of substances such as enzymes, antibiotics, and recombinant proteins.

Further, the production method of the invention can also be applied to production of a chemical product, a dairy product, a pharmaceutical product, a food product, or a brewery product. Examples of the chemical product include organic acids, amino acids, and nucleic acids. Examples of the dairy product include low fat milk. Examples of the food product include lactic acid drink, and examples of the brewery product include beer and soju. Further, the enzymes, antibiotics, recombinant protein, or the like that are produced according to the production method of the invention can be also used for a pharmaceutical product.

(F) Culturing

For producing a chemical by continuous fermentation, batch culture or fed-batch culture may be conducted at an initial stage of culture to increase the density of microorganisms, followed by initiating continuous fermentation (withdrawal of culture liquid). Alternatively, the density of microorganisms may be increased followed by seeding a high density of microorganisms, thereby initiating culture and simultaneously carrying out continuous fermentation. For producing a chemical by continuous fermentation, supply of the starting culture liquid and withdrawal of the culture may be initiated at a suitable stage. The time of initiating supply of the starting culture liquid and the time of initiating withdrawal of the culture liquid may not always be the same. Supply of the starting culture liquid and withdrawal of the culture liquid may be conducted continuously or intermittently.

Nutrients necessary for growth of the microorganism may be added to the culture liquid so that the microorganism grows continuously. For attaining efficient productivity, the density of microorganisms or culture cells in the culture liquid is preferably kept high in a range that the environment of the culture liquid is not made unsuitable for growth of the microorganisms or culture cells to cause a high death rate. By way of example, for D-lactic acid fermentation using SL lactobacillus, the microorganisms or culture cells in the culture liquid kept at a density of not lower than 5 g/L in dry weight thereby makes it possible to obtain good production efficiency.

For producing a chemical by continuous fermentation, when sugars are used as feedstock, concentration of sugars in culture liquid is preferably maintained at the level of 5 g/L or less. The reason that the concentration of sugars in the culture liquid is kept preferably at 5 g/L or less is that the loss of sugars upon withdrawal of the culture liquid can be minimized.

The microorganisms or culture cells are cultured usually at pH 3 to 8 at a temperature in the range of 20°C to 60°C. The pH of the culture liquid is adjusted to a predetermined value usually in the range of pH 3 to 8 with an inorganic or organic acid, an alkaline substance, urea, calcium carbonate, ammonia gas, or the like. When it is necessary to increase the supply rate of oxygen, it is possible to employ means for adding oxygen to air to maintain an oxygen concentration not lower than 21%, pressurizing the culture liquid, increasing the stirring rate, or enhancing aeration.

For operation of continuous fermentation, it is preferable to monitor the microorganism concentration in a tank used for fermenting microorganisms. Measurement of microorganism concentration may be achieved by collecting and measuring a sample. However, it is preferable to monitor continuously the changing state of microorganism concentration by installing a sensor for microorganism concentration such as MLSS detector in a tank used for fermenting microorganisms.

For producing a chemical by continuous fermentation, the culture liquid, microorganisms or culture cells can be withdrawn as necessary from the fermentation tank. Because the separation membrane is easily clogged, for example, when the density of the microorganisms or culture cells in the fermentation tank becomes too high, such clogging can be prevented by withdrawal. The performance of production of a chemical may vary depending on the density of the microorganisms or culture cells in the fermentation tank, and the productive performance can be maintained by withdrawing the microorganisms or culture cells with the productive performance as an indicator.

For producing a chemical by continuous fermentation, the number of fermentation tanks is not limited as long as the operation of continuous culture during which fresh microorganisms capable of fermentation production are grown is carried out by a continuous culture method wherein the microorganisms are grown and simultaneously a product is formed. In the method of producing a chemical by continuous fermentation, it is preferable for control of culture that the operation of continuous culture is carried out in a single fermentation tank. However, a plurality of fermentation tanks may be used for reasons such as a small capacity of the fermentation tank. In this case, plural fermentation tanks can be connected via pipes in parallel, or in series, in continuous fermentation to achieve high productivity of the fermentation product.

2. Membrane separation step

(A) Separation membrane

Next, explanations are given for the separation membrane that is used for membrane separation step of the method for producing a chemical.

The separation membrane can be any one of an organic membrane and an inorganic membrane. Since backwashing or washing by immersion in chemical liquid is carried out for washing the separation membrane, the separation membrane preferably has durability therefor.

From the viewpoint of separation performance, water permeability, and also fouling resistance, an organic polymer compound can be preferably used. Examples thereof include a polyethylene resin, a polypropylene resin, a polyvinyl chloride resin, a polyvinylidene fluoride resin, a polysulfone resin, a polyether sulfone resin, a polyacrylonitrile resin, a cellulose resin and a cellulose triacetate resin. It may be a resin mixture containing these resins as the major component.

A polyvinyl chloride resin, a polyvinylidene fluoride resin, a polysulfone resin, a polyether sulfone resin, and a polyacrylonitrile resin, which can be easily formed into a film from a solution and have excellent physical durability and chemical resistance, are preferable. Further, a polyvinylidene resin or a resin containing the same as the major component is more preferably used because they characteristically have both the chemical strength (chemical resistance, in particular) and physical strength.

As used herein, a homopolymer of vinylidene fluoride is preferably used as the polyvinylidene fluoride resin. As the polyvinylidene fluoride resin, a copolymer of vinylidene fluoride and a vinyl monomer copolymerizable therewith can also be preferably used. The vinyl monomer copolymerizable with vinylidene fluoride can be exemplified by tetrafluoroethylene, hexafluoropropylene and ethylene chloride trichloride.

More preferably, the separation membrane is a hollow fiber membrane containing fluoro resin-based polymer, and it is a hollow fiber membrane having both three-dimensional mesh structure and spherical structure and hydrophilicity by containing a hydrophilic polymer including, in the three-dimensional mesh structure, at least one selected from fatty acid vinyl ester, vinyl pyrrolidone, ethylene oxide, and propylene oxide, or cellulose ester.

As used herein, the three-dimensional mesh structure indicates a structure in which solid matters are three dimensionally dispersed in mesh state. The three-dimensional mesh structure includes micropores and voids that are separated by solid matters for constituting the mesh.

Further, the spherical structure indicates a structure in which many spherical or almost spherical solid matters are connected to each other either directly or via solid matters in stripe pattern.

Further, it is not particularly limited if it has both the spherical structure layer and three-dimensional mesh structure layer. However, a laminate structure including the spherical structure layer and three-dimensional mesh structure layer is preferable. In general, when layers are stacked in a multi-layer form, permeability is lowered at interface of each layer as layers are squeezed into each other to have high density. When the layers are not squeezed into each other, although permeability is not lowered, interface peel strength is lowered. Thus, considering interface peel strength and permeability of each layer, it is preferable to have a small laminate number of the spherical structure layer and three-dimensional mesh structure layer. More particularly, a total of two layers including one layer of spherical structure layer and one layer of three-dimensional mesh structure layer are laminated.

Further, the separation membrane may contain a layer other than the spherical structure layer and three-dimensional mesh structure layer, for example, a support layer such as porous base. The porous base material is, although not particularly limited, an organic material, and an inorganic material, and organic fibers are desirably used as light weightness can be achieved. The porous base material is more preferably a woven or nonwoven fabric prepared from organic fibers such as cellulose fibers, cellulose acetate fibers, polyester fibers, polypropylene fibers, and polyethylene fibers.

Top and bottom and also inside and outside arrangement in the spherical structure layer and three-dimensional mesh structure may be varied depending on filtration mode. Because the three-dimensional mesh structure is responsible for separation function and the spherical structure layer is responsible for physical strength, it is preferable that the three-dimensional mesh structure is arranged on a side of subject to be separated. In particular, to inhibit decrease in permeability caused by adhesion of contaminating substances, it is preferable that the three-dimensional mesh structure responsible for separation function is placed on the outermost surface layer of the separation subject side.

The average pore diameter may be suitably determined depending on purpose or environment for use, and somewhat small diameter is preferable. Generally, it is 0.01 $\mu$m or more but the same or less than 1 $\mu$m. When the average pore diameter of hollow fiber membrane is less than 0.01 $\mu$m, components such as sugar and protein or contaminating components such as their aggregates block the pores, making it difficult to have stable operation. Considering a balance with water permeability, it is preferably 0.02 $\mu$m or more, and more preferably 0.03 $\mu$m or more. Further, when it is more than 1 $\mu$m, removal of contaminating components from pores, which is achieved by shear force created by smoothness of membrane surface and flow on membrane surface or physical washing such as back washing or air scrubbing, becomes insufficient, making it difficult to achieve stable operation. Further, when the average pore diameter of the hollow fiber membrane is close to the size of microorganisms or culture cells, they may block the pores by themselves. Further, there can be a case in which cell debris are generated by death of part of microorganisms or culture cells in fermentation liquid, and thus to avoid clogging of the hollow fiber membrane by debris, the average pore diameter is preferably 0.4 $\mu$m or less. The operation can be performed more preferably when it is 0.2 $\mu$m or less.

Herein, the average pore diameter can be determined by measuring the diameters of plural pores that are observed under a scanning electron microscope at a magnification of 10,000 or more, and then averaging the measured diameters. Preferably, it is determined by selecting 10 or more, preferably 20 or more, pores at random, measuring the diameters of the selected pores, and number-averaging the measured diameters. When the pore is not circular, the average pore size can be preferably determined by a method of determining a circle having area equivalent to that of the pore, that is, an equivalent circle with an image processor or the like, and assuming that the diameter of the equivalent circle is the diameter of the pore.

(B) Separation condition

The transmembrane pressure difference for filtering treatment of fermentation liquid of microorganisms or culture cells with a separation membrane in a membrane module can be on condition that does not allow easy clogging by microorganisms and culture cells, and culture medium components. For example, the filtration treatment can be performed with a transmembrane pressure difference in the range of 0.1 to 20 kPa. Preferably, the transmembrane pressure difference is in the range of 0.1 to 10 kPa. More preferably, the transmembrane pressure difference is in the range of 0.1 to 5 kPa. When the transmembrane pressure difference is within the above range, clogging by microorganisms (prokaryotes, in particular) and culture medium components and decrease in the amount of permeable water are suppressed so that problems occurring during continuous fermentation operation can be efficiently inhibited.

As for the driving force for filtration, transmembrane pressure difference in separation membrane can be generated by siphon with liquid level difference between fermentation liquid and porous membrane treated water (i.e., water head difference) or cross flow circulating pump. In addition, for providing a driving force for filtration, a suction pump may be installed at the side of separation membrane treated water. Further, when a cross flow circulating pump is used, the transmembrane pressure difference can be controlled based on suction pressure. The transmembrane pressure difference can be also controlled based on pressure of gas or liquid for applying pressure on the side of fermentation liquid. When such pressure control is performed, the pressure difference between the pressure at the side of fermentation liquid and the pressure at the side of porous membrane treated water can be taken as transmembrane pressure difference and used for control of transmembrane pressure difference.

(C) Type of separation membrane

Shape of a separation membrane may be any one of plane membrane, hollow fiber membrane, and spiral type. When it is a hollow fiber membrane module, any of external pressure type and internal pressure type may be adopted.

Specifications such as length of separation membrane module, filling ratio, and type of separation membrane may be the same or varied. However, when the filling ratio is changed, for example, cross flow flux is different for each module, and a phenomenon in which the separation membrane washing effect by shear force generated by cross flow motion is different may occur. Further, the filtration rate of a module needs to be set separately, and thus part number increases, making the inventory managing more cumbersome. Thus, from the viewpoint of production management, specifications are preferably the same.

3. Step for washing separation membrane

The method for producing a chemical may include a step for washing separation membrane. The washing step preferably includes, although not particularly limited, in addition to removal of precipitates such as microorganisms on separation membrane by shear force of cross flow on surface at primary side of the separation membrane according to intermittent filtration treatment which includes repeating filtration treatment and filtration-stop treatment, washing the separation membrane by backwashing or immersing in backwashing liquid. When the intermittent filtration treatment is carried out by using plural separation membrane modules, it is preferable that the filtration-stop treatment by plural separation membrane modules that are arranged in parallel or in series is controlled to have no overlap so that the filtration is not stopped entirely.

Herein, washing the separation membrane indicates washing by passing the washing liquid from secondary side to primary side of the separation membrane after stopping the filtration (i.e., backwashing), a method of immersing at separation membrane part after passing washing liquid from secondary side to primary side of the separation membrane, or a method of washing by passing washing liquid containing a reducing agent from primary side to secondary side of the separation membrane or from secondary side to primary side of the separation membrane after stopping filtration and performing backwashing by supplying, from secondary side to primary side of the separation membrane, washing liquid containing an oxidizing agent.

When the separation membrane is washed with filtration stop, gas may be simultaneously supplied to the module either continuously or intermittently. Further, for backwashing of the separation membrane, the cross flow may or may not be present. When backwashing is carried out while the cross flow is present, the backwashing can be carried out with the pressure which is higher than the total of cross flow pressure and pressure difference between separation membranes.

As described herein, the backwashing indicates a method of removing contaminating substances on membrane surface by passing washing liquid from filtration liquid side, i.e., secondary side, to the fermentation liquid side, i.e., primary side of the separation membrane. Backwashing can be carried out by using water or washing liquid. As for the washing liquid, water containing alkali, acid, oxidizing agent, or reducing agent may be used to the extent that the fermentation is not significantly inhibited. Herein, examples of the alkali include sodium hydroxide and calcium hydroxide. Examples of the acid include oxalic acid, citric acid, hydrochloric acid, and nitric acid. Examples of the oxidizing agent include hypochlorite salt and peroxide. Examples of the reducing agent include an inorganic reducing agent such as sodium hydrogen sulfite, sodium sulfite, and sodium thiosulfate.

Further, since the backwashing is performed for preventing large transmembrane pressure difference between separation membranes, it is preferably carried out periodically with appropriate time interval. Since fermentation is continuously performed for continuous fermentation, it is necessary to add a pH controlling liquid whenever pH change occurs in accordance with fermentation, and thus the pH control liquid needs to be added continuously. Although it may be considered to add alkali or acid to a backwashing liquid, which is then used for pH control of fermentation liquid, it is difficult to limitedly say that backwashing is performed at the timing requiring pH control, and therefore it is not suitable for an application related to pH control.

Further, it is difficult to limitedly say that the alkali or acid added for washing the separation membrane for backwashing is the same as the alkali or acid that is required for pH control at that time, and there can be also a case in which, although an alkali is added for backwashing, an acid needs to be added for pH control.

Further, since the backwashing includes permeation from secondary side to primary side of the separation membrane, the alkali or acid preferably contains no solid matter. However, for pH control, it is only required to be soluble after being added to a fermentation liquid, and thus an alkali or an acid in slurry phase may be used.

For example, when a chemical obtained by fermentation is lactic acid, it is necessary to use an alkali for pH neutralization, which shifts toward an acid side in accordance with lactic acid production, to maintain an optimum pH for fermentation. However, as fermentation rate becomes faster in continuous fermentation, a great amount of an alkali needs to be added. When calcium hydroxide is used as a neutralizing agent, it is present as a solid since it does not dissolve at concentration of about 0.01 N or higher, and therefore it is not suitable as a backwashing liquid. For such reasons, neutralization is performed with a calcium hydroxide solution with concentration of about 0.01 N or less, but in such case, a larger amount of pH control liquid needs to be added, which eventually leads to diluted fermentation liquid, yielding lower chemical concentration. As a result, there is a problem that extra energy is required for performing evaporation or the like during post treatment to obtain a chemical from the fermentation liquid.

When pH of fermentation liquid is out of the optimal range even for a temporal period, the fermentation output may be lowered and activity of microorganisms may be impaired during that period. For such reasons, even when an alkali or an acid is added for backwashing, having both functions of backwashing liquid and pH controlling liquid is not obtained. Thus, to control pH of fermentation liquid within an appropriate range, it is necessary to have separately a control device for pH control.

When backwashing liquid contains an oxidizing agent, there is a possibility that the oxidizing agent remains in the separation membrane module and pipes at filtration side, i.e., a secondary side, after washing. Thus, it is possible that, after backwashing, an aqueous solution containing a reducing agent is passed through from the primary side to the secondary side. At that time, concentration of a reducing agent can be between 1 ppm and 5000 ppm. Preferably, it is from one to five times or so the theoretical concentration required for reducing neutralization, compared to the presumably remaining oxidizing agent. Further, the period for filtering an aqueous solution containing a reducing agent is determined based on the period of backwashing using an oxidizing agent. It is also possible that, considering an influence on microorganisms or the like, backwashing using plural oxidizing agents is performed and then washing with the reducing agent is performed, if required.

Further, with respect to the time for filtering water containing a reducing agent and injection rate, it is preferably performed until the oxidizing agent in the separation membrane module or the like is neutralized by reduction. For example, when sodium hypochlorite is used as an oxidizing agent, it is preferably performed until the free chlorine concentration in secondary pipe at filtration side is 0.1 ppm or so. Examples of the method for measuring free chlorine concentration include DPD method, electric current method, and a method using spectrophotometer. For the measurement, water is suitably collected and free chlorine concentration is measured by DPD method or electric current method. Using a continuous automatic measurement device equipped with spectrophotometer, free chlorine concentration is measured. According to the measurement, free chlorine concentration is monitored and the time for filtering water added with a reducing agent is determined.

With regard to the washing liquid within the range that the effect of the invention is not inhibited as described herein, for sodium hypochlorite, for example, it is preferable to use a washing liquid having effective chlorine concentration of from 10 to 5000 ppm. For sodium hydroxide and calcium hydroxide, for example, a washing liquid with pH of from 10 to 13 is preferably used. Separation membrane damage or bad influence on microorganisms may occur at a concentration which is higher than the above range. On the other hand, at a concentration which is lower than the above range, there may be a decrease in the membrane washing effect.

The backwashing liquid can be also used at high temperature. In addition, the backwashing rate of the backwashing liquid is preferably from 0.5 times to 10 times the membrane filtration rate. More preferably, it is from 1 time to 5 times the membrane filtration rate. When the backwashing rate is the same or less than 10 times the membrane filtration rate, a possibility of having separation membrane damage can be lowered. Further, when it is the same or higher than 0.5 times the membrane filtration rate, the washing effect can be obtained at sufficient level.

Backwashing period with backwashing liquid may be determined based on transmembrane pressure difference and a change in transmembrane pressure difference. The backwashing period is in the range of 0.5 times to 12 times per hour. More preferably, it is in the range of 1 time to 6 times per hour. When the backwashing period is greater than the range, damages may occur on a separation membrane, and thus time for filtration is shortened. On the other hand, when it is smaller than the range, the washing effect may not be obtained at sufficient level.

Backwashing time with backwashing liquid may be determined based on backwashing period, transmembrane pressure difference, and a change in transmembrane pressure difference. The backwashing time is in the range of 5 sec to 300 sec per washing. More preferably, it is in the range of 30 sec to 120 sec per washing. When the backwashing time is longer than the range, damages may occur on a separation membrane. On the other hand, when it is shorter than the range, the washing effect may not be obtained at sufficient level.

For backwashing, it is also possible to immerse the separation membrane in backwashing liquid after stopping the filtration first. The immersion time may be determined based on immersion washing period, transmembrane pressure difference, and a change in transmembrane pressure difference. The immersion time is preferably in the range of from one minute to 24 hours per immersion, and more preferably in the range of from 10 minutes to 12 hours per immersion.

For a continuous fermentation apparatus, it is also preferable that separation membranes are arranged in multiple series and, when the separation membranes are subjected to immersion washing with backwashing liquid, conversion is made so as to have immersion washing of only part of the series, and thus the filtration is not stopped entirely.

With regard to the washing liquid storage tank (i.e., washing liquid tank), pumps for supplying washing liquid, and pipes from the washing liquid storage tank to module, and valves, those having excellent chemical resistance can be used. Although injection of backwashing liquid can be manually carried out, it is preferable that injection is carried out, after installing a device for controlling filtration and back washing, by automatically controlling the filtration pump, filtration side valve, washing liquid supply pump, and washing liquid supply valve by using a timer or the like.

<Apparatus for chemical production>

The continuous fermentation apparatus used for the embodiment of the invention is described in view of drawings. The continuous fermentation apparatus described below is an exemplary apparatus for carrying out the method for producing a chemical described above. Thus, regarding the constitutions of the apparatus for carrying out the production method, explanation of the constitutions which have been described already in the section of production method may be omitted.

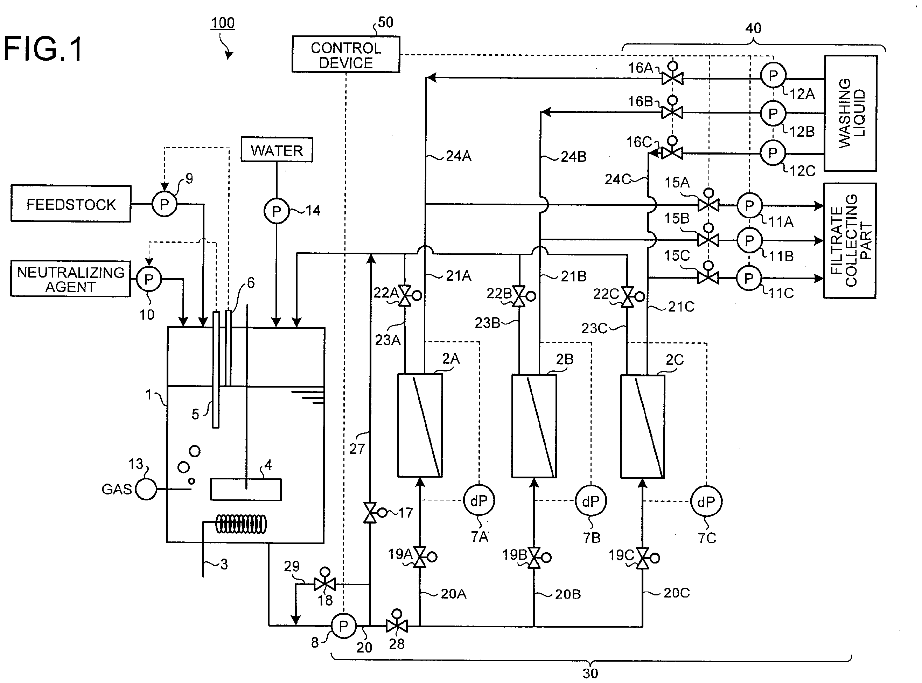

FIG. 1 is a schematic diagram illustrating a membrane separation type continuous fermentation apparatus. A membrane separation type continuous fermentation apparatus 100 is equipped with a fermentation tank 1 for converting fermentation feedstock to a fermentation liquid containing a chemical by fermentation culture of microorganisms or the like and a separation membrane unit 30 for collecting a chemical as a filtered liquid by filtering the fermentation liquid converted in the fermentation tank 1 and for returning non-filtered liquid to the fermentation tank 1. In the separation membrane unit 30, three separation membrane modules 2A, 2B, and 2C are arranged in parallel.

The fermentation tank 1 is equipped with a temperature control device 3 for controlling the temperature inside the fermentation tank 1, a stirring device 4 for stirring fermentation liquid in the fermentation tank 1, a pH sensor and control device 5 for detecting pH of culture liquid and controlling a neutralizing agent supply pump 10 according to detection result so that pH of the culture liquid is maintained within the set pH region, a level sensor and control device 6 for controlling a culture medium supply pump 9 and a water supply pump 14 for detecting liquid level in the fermentation tank 1 and maintaining the liquid level within the set range, and a gas supply device 13 for supplying gas to the fermentation tank 1.

Feedstock and microorganisms or culture cells are added to the fermentation tank 1. The fermentation step progresses in the fermentation tank 1. First, by means of the culture medium supply pump 9, feedstock is added from the feedstock tank to the fermentation tank 1. The neutralizing agent supply pump 10 is connected to the neutralizing agent bath for storing a neutralizing agent, and in the neutralizing agent bath, an acidic aqueous solution or an alkaline aqueous solution suitably selected depending on feedstock and microorganisms or culture cells that are used are contained. By operating the neutralizing agent supply pump 10 to add the neutralizing agent to the fermentation tank 1, the pH sensor and control device 5 controls pH of the culture liquid at desired pH. As the pH of the culture liquid is maintained within a certain range, fermentation production can be performed with high productivity. The neutralizing agent, i.e., an acidic aqueous solution or an alkaline aqueous solution, corresponds to the pH controlling liquid.

The temperature control device 3 is equipped with a temperature sensor for detecting temperature, a heating part, a cooling part, and a control part. The temperature control device 3 measures the temperature inside the fermentation tank 1 and, according to detection result, controls the temperature of the heating part and the cooling part by the control part so that the temperature exhibits a value within a certain range. By doing so, the temperature of the fermentation tank 1 is maintained at constant level, allowing high concentration of the microorganisms.

Water may be added directly or indirectly to the fermentation tank 1. The water supply pump 14 directly supplies water to the fermentation tank 1. Indirect water supply includes supply of feedstock and addition of a pH controlling agent, or the like. Materials to be added to a continuous fermentation apparatus are preferably sterilized to prevent contamination by contaminants and to achieve efficient fermentation. For example, the culture medium may be sterilized by heating after mixing the culture medium materials. In addition, the water to be added to culture medium, pH controlling liquid, and a fermentation tank may be sterilized by filtering through a sterilizing filter, if necessary.

The level sensor and control device 6 is equipped with a sensor for detecting liquid level in the fermentation tank 1, and a control device. By controlling the culture medium supply pump 9 and the water supply pump 14 based on the detection result obtained by the sensor, the control device controls liquid amount introduced to the fermentation tank 1, and as a result, the liquid level in the fermentation tank 1 is maintained within a certain range.

For aerobic fermentation, gas is introduced to the fermentation tank 1 by means of the gas supply device 13 and fermentation is performed by dissolving oxygen in fermentation liquid. However, for continuous fermentation, to collect a chemical from filtrate and to maintain or return simultaneously the microorganisms or culture cells in concentrated liquid to fermentation culture liquid when microorganisms or culture cells are filtered using a separation membrane module 2, the fermentation liquid is subjected to cross flow circulation in the separation membrane module 2. By supplying gas to a liquid transporting line for circulation or the separation membrane module 2, oxygen can be dissolved in fermentation liquid at a place different from the fermentation tank 1 and the microorganisms or the like precipitated on the surface of the separation membrane can be removed by shear force of the gas.

Herein, with regard to the gas, gas containing oxygen is required for aerobic fermentation. It may be supplied in the form of pure gas or gas having no adverse effect on fermentation, for example, air, nitrogen, carbon dioxide, methane, or gas with adjusted oxygen concentration by adding mixture gas containing those gases. Meanwhile, for anaerobic fermentation, if it is necessary to decrease the oxygen supply rate, it is possible to supply mixture obtained by mixing air with gas containing no oxygen such as carbon dioxide, nitrogen, methane, and argon.

A gas supply source may be a device capable of compressing gas and then supplying the compressed gas at constant pressure, or a tank capable of supplying gas at constant pressure in which the gas has been already compressed. Compressed gas supplied by means of a gas cylinder, a blower, a compressor, or a pipe can be used.

On the pipe from the gas supply source to a gas supply outlet, a flow meter or the like is installed so as to allow measurement of gas supply amount. In addition, by installing a valve or the like on the pipe, the supply flow amount is controlled. The valve is to control the flow amount of gas, and by installing an automatic valve, the gas supply can be made intermittently. Gas supply can be made manually by using a valve. However, it is preferable that, by installing a device for controlling gas supply amount, gas is supplied by automatically controlling a filtration pump, a filtration side valve, a gas supply valve, and a flow meter by means of a timer or the like. However, if the flow rate of gas to be supplied can be determined and the flow rate thereof can be controlled without installing the flow meter, the valve, or the control device, it is not particularly limited.

On the pipe from the gas supply source to the gas supply outlet, a sterilizing device or a sterilizing filter is preferably installed to prevent incorporation of unwanted bacteria to a fermentation system.

The gas supply outlet is only required to supply gas from the gas supply source to primary side of the separation membrane module 2. The gas supply outlet may be formed at bottom part of the separation membrane module 2, or may be formed on a pipe 20 which connects the separation membrane module 2 to the fermentation tank 1. When fermentation liquid is transported from the fermentation tank 1 to the separation membrane module 2 by means of a circulation pump 8, the gas supply outlet may be formed between fermentation liquid and the circulation pump 8 or between the circulation pump 8 and the separation membrane module 2.

The gas supply outlet can have any size if it allows supply of gas supply amount and no clogging is caused by fermentation liquid. To prevent incorporation of unwanted bacteria to the fermentation system, a sterilizing filter or the like may be installed.

Further, when a gas supply line is installed for each of the separation membrane modules 2A, 2B, and 2C, gas supply can be made for each of the separation membrane modules 2A, 2B, and 2C also. It is also possible that, according to an effect of removing microorganisms or the like precipitated on the surface of the separation membrane by shear force of gas, gas can be intermittently supplied to reduce the use amount of gas.

The separation membrane unit 30 is equipped with the separation membrane module 2, the circulation pump 8 for transporting fermentation liquid to the separation membrane module 2, a separation membrane washing device 40 for performing backwashing of the separation membrane module 2, and a control device 50 for controlling each part of the separation membrane unit 30.

The separation membrane module 2 is equipped with a number of hollow fiber membranes. Regarding the separation membrane module 2, it is preferable that fermentation liquid be evenly transported, and to achieve even transport, liquid transporting resistance is small compared to liquid transporting pressure, depending on viscosity of fermentation liquid to be transported and length and thickness of the pipe of the liquid transporting line. Although three separation membrane modules 2A, 2B, and 2C are arranged in parallel, the number of the separation membrane modules 2 is not particularly limited as long as plural modules are used. The number of the series of the separation membrane module 2 is preferably determined in consideration of an operation mode of the separation membrane module 2 described below and also specifications of a circulation pump to be used.

Specifications of the separation membrane modules 2A, 2B, and 2C including length, filling ratio, and type of separation membrane may be the same or varied. However, when the filling ratio is changed, for example, cross flow flux is different for each separation membrane module 2, and a phenomenon in which separation membrane washing effect by shear force generated by cross flow motion is different may occur. Further, the filtration rate of the separation membrane module 2 needs to be set separately, and thus part number increases, making the inventory managing more cumbersome. Thus, from the viewpoint of production management, specifications of the separation membrane modules 2A, 2B, and 2C are preferably the same.

By means of the circulation pump 8, fermentation liquid in the fermentation tank 1 is transported to the separation membrane modules 2A, 2B, and 2C via the pipe 20. Between the circulation pump 8 and the separation membrane modules 2A, 2B, and 2C, circulating valve 28 is installed. The pipe 20 is branched to a pipe 20A, a pipe 20B, and a pipe 20C, and on the pipe 20A, the pipe 20B, and the pipe 20C, a valve 19A, a valve 19B, and a valve 19C are installed, respectively.

On the pipe 20, which is a supply line for fermentation liquid from the fermentation tank 1 to the separation membrane module 2, a pipe 27 as a bypass for circulating to the fermentation tank 1 without intervention of the separation membrane module 2 and a valve 17 and a pipe 29 as a bypass for circulating to the line introduced with the circulation pump 8 and a valve 18 are included. By forming a circulating bypass line, for a case in which a part of the separation membrane module 2 is stopped when filtering property is deteriorated or the like, the cross flow rate corresponding to the separation membrane module 2 in stop mode can be flown in the bypass, and thus fluctuation in cross flow pressure can be suppressed.

The filtrate filtered by each of the separation membrane modules 2A, 2B, and 2C is transported to a filtrate collecting part via a pipe 21A, a pipe 21B, and a pipe 21C. On the pipe 21A, the pipe 21B, and the pipe 21C, a filtration valve 15A, a filtration valve 15B, and a filtration valve 15C, and a filtration pump 11A, a filtration pump 11B, and a filtration pump 11C are installed, respectively. Further, in the separation membrane modules 2A, 2B, and 2C, pressure difference sensors 7A, 7B, and 7C are installed for measuring pressure difference between the primary side to which fermentation liquid is supplied and the secondary side at which filtrate is filtered.

With the control device 50 which opens the circulating valve 28, a valve 19, and a filtration valve 15 and allows transport of fermentation liquid to the separation membrane modules 2A, 2B, and 2C with an aid of the circulation pump 8, the chemical as fermentation product is recovered by filtration. At the time of filtration, it is possible that the pressure difference between the primary side and the secondary side is measured by using a pressure difference sensor 7 and filtration suction is performed by driving a filtration pump 11. However, even without installing the pressure difference sensor 7 and the filtration pump 11, the filtrate can be collected.

The fermentation liquid not filtered by the separation membrane modules 2A, 2B, and 2C (i.e., non-filtered liquid) is returned to the fermentation tank 1 via a pipe 23A, a pipe 23B, and a pipe 23C. On the pipe 23A, the pipe 23B, and the pipe 23C, a valve 22A, a valve 22B, and a valve 22C are installed, respectively.

The separation membrane washing device 40 is equipped with a washing liquid tank for storing washing liquid, a pipe 24A, a pipe 24B, and a pipe 24C for transporting the washing liquid to each of the separation membrane modules 2A, 2B, and 2C, and a supply pump 12A, a supply pump 12B, and a supply pump 12C. Further, on the pipe 24A, the pipe 24B, and the pipe 24C, a washing liquid valve 16A, a washing liquid valve 16B, and a washing liquid valve 16C are installed, respectively.

With regard to the washing liquid tank, a supply pump 12, a pipe 24 from the washing liquid tank to the separation membrane module 2, and a washing liquid valve 16, those having excellent chemical resistance can be used. Although injection of backwashing liquid can be manually carried out, it is preferable that injection be carried out by automatically controlling the filtration pump 11, the filtration valve 15, the supply pump 12, and the washing liquid valve 16 by using a timer or the like with an aid of the control device 50.

Conditions for backwashing may be the same or varied separately for the separation membrane modules 2A, 2B, and 2C. For example, it is possible that the pressure difference between separation membranes is measured by the pressure difference sensor 7 and backwashing rate is increased for the separation membrane module 2 having high clogging (i.e., high pressure difference) or backwashing time is elongated. On the other hand, it is also possible that backwashing rate is decreased for the separation membrane module 2 having little clogging, or backwashing time is shortened and filtration time is elongated.

In addition, by varying the pressure of fermentation liquid supplied to the primary side of the separation membrane module 2, filtering performance of the separation membrane can be maintained at good level. Also, a turbulent flow region can be created locally by varying the discharge pressure of the circulation pump 8 so that shear force of the fermentation liquid of cross flow is increased and precipitates such as microorganisms precipitated on the surface of the separation membrane can be removed.

Varying the discharge pressure of the circulation pump 8 may be continuous variation. In general, operation is made with almost constant discharge pressure of the circulation pump 8. However, by modifying only the set time according to manipulating the control valve or the like, the discharge pressure of the circulation pump 8 may be varied intermittently.

When variation in the discharge pressure of the circulation pump 8 is excessively small, the effect of removing precipitates is small. On the other hand, when the pressure variation is excessively high, a leak from a connecting part may be caused due to hunting of a liquid transporting pipe. For such reasons, the degree of the pressure variation of the circulation pump 8 is preferably between 3% and 20% compared to discharge pressure.

When the discharge pressure of the circulation pump 8 is varied, gas can be introduced to fermentation liquid to be supplied by introducing gas simultaneously to the liquid transporting line for cross flow circulation, for example, to the pipe 20 and the separation membrane module 2, and shear force can be strengthened by the gas introduced to fermentation liquid. As a result, the effect of removing microorganisms or the like that are precipitated on the surface of the separation membrane can be further enhanced.

With the control device 50 allowing opening of the washing liquid valve 16 and transporting the washing liquid via the supply pump 12 to filtrate side as the secondary side of the separation membrane module 2, backwashing of the separation membrane module 2 is carried out. At that time, the valve 19 on the pipe 20 for supplying the fermentation liquid to the separation membrane module 2 is closed while the valve 22 on a pipe 23 for returning the non-filtered liquid to the fermentation tank 1 is open. In addition, in order to prevent incorporation of washing liquid to the filtrate collecting part, control is made to close the filtration valve 15.

According to the membrane separation type continuous fermentation apparatus 100 of the first embodiment, the fermentation liquid containing fermentation product is filtered by the separation membrane module 2 to be isolated as microorganisms or culture cells and fermentation product, which are then taken out of an apparatus system. In addition, the isolated microorganisms or culture cells are returned to the fermentation tank 1 and stay in the apparatus system, and thus concentration of the microorganisms is maintained at high level in the apparatus system. As a result, fermentation production with high productivity can be achieved.

In the separation membrane unit 30, precipitates on the surface of the separation membrane can be removed by shear force of cross flow motion. It is preferable that, by performing intermittent filtration in which filtration treatment and filtration-stop treatment are alternately repeated and, particularly, increasing shear force of cross flow motion during filtration-stop treatment in which filtration is stopped, precipitates on the surface of the separation membrane be removed. For example, for intermittent filtration in which filtration treatment for nine minutes and filtration-stop treatment for one minute are repeated, during nine minutes of filtration, feedstock is added to a fermentation tank in the same amount as the fermentation liquid reduced by filtration, but during one minute of filtration-stop, the entire amount of fermentation liquid is returned to the fermentation tank 1 by the circulation pump 8 so that the fermentation liquid is not reduced and the feedstock is not added to the fermentation tank. Thus, in the membrane separation type continuous fermentation apparatus 100, when filtration treatment for nine minutes and filtration-stop treatment for one minute are performed at the same timing using the separation membrane modules 2A, 2B, and 2C, the feedstock is added during filtration, but it is not added during the filtration-stop treatment. When addition of feedstock is intermittent, concentration of the feedstock is not stable in the fermentation tank 1, and it may jeopardize stable fermentation. For such reasons, in the first embodiment, it is effective to control such that the separation membrane modules 2A, 2B, and 2C do not simultaneously have filtration-stop and the filtration-stop treatments of the separation membrane modules 2 are not overlapped so that an even filtering amount can be obtained.

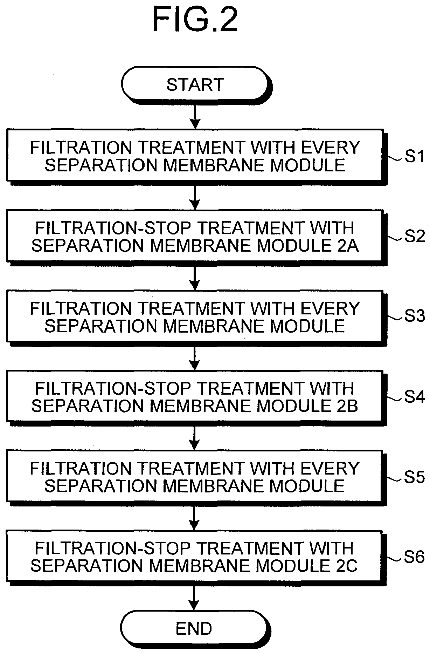

The intermittent filtration treatment will be described in view of FIG. 2. FIG. 2 is a flowchart for describing the intermittent filtration treatment. When plural separation membrane modules 2A, 2B, and 2C are used for intermittent filtration treatment, it is preferable to perform the intermittent filtration treatment by controlling the timing of the filtration-stop treatment of the separation membrane modules 2A, 2B, and 2C. Controlling the timing of the filtration-stop treatment means that, for example, the filtration-stop treatment of at least one of the separation membrane modules 2 is performed during the filtration treatment of other separation membrane modules 2. Preferably, control is made such that the filtration-stop treatments of the separation membrane modules 2A, 2B, and 2C are not overlapped with each other. When intermittent filtration is made such that the filtration-stop treatments of the separation membrane modules 2A, 2B, and 2C are not overlapped with each other, the filtration treatment is performed first with all of the separation membrane modules 2A, 2B, and 2C (Step S1). In order to perform the filtration treatment with all of the separation membrane modules 2A, 2B, and 2C, the filtration valves 15A, 15B, and 15C are open, and with driving of the filtration pumps 11A, 11B, and 11C, fermentation liquid is supplied to the separation membrane modules 2A, 2B, and 2C by means of the circulation pump 8 for filtration. Further, in every step described below, the circulating valve 28, the valve 19A, the valve 19B, and the valve 19C, the valve 22A, the valve 22B, and the valve 22C are all in an open state, and the fermentation liquid not filtered by the separation membrane modules 2A, 2B, and 2C is subjected to cross flow to the fermentation tank 1.

After a certain period of time (for example, two minutes later), the separation membrane module 2A is driven for the filtration-stop treatment (Step S2). When the separation membrane module 2A is driven for the filtration-stop treatment and remaining separation membrane modules 2B and 2C are driven for filtration treatment, the filtration valves 15B and 15C are open, the filtration valve 15A is closed, the filtration pumps 11B and 11C are driven and the filtration pump 11A is stopped, and also fermentation liquid is supplied to the separation membrane modules 2A, 2B, and 2C by means of the circulation pump 8. Accordingly, filtration occurs only in the separation membrane modules 2B and 2C. The separation membrane module 2A has the filtration-stop treatment and the precipitates on the membrane are removed by cross flow fermentation liquid in the separation membrane module 2A.

After a certain period of time (for example, one minute later), the filtration-stop treatment by the separation membrane module 2A is terminated and filtration treatment is carried out using all of the separation membrane modules 2A, 2B, and 2C (Step S3). By switching the filtration valve 15A to an open state and driving the filtration pump 11A, the filtration treatment is performed in all of the separation membrane modules 2A, 2B, and 2C.

After a certain period of time (for example, two minutes later), the filtration-stop treatment by the separation membrane module 2B is performed (Step S4). When the filtration-stop treatment is performed with the separation membrane module 2B and remaining separation membrane modules 2A and 2C are used for filtration treatment, by switching the filtration valve 15B to a closed state and stopping the filtration pump 11B, filtration occurs only in the separation membrane modules 2A and 2C, and the filtration-stop treatment occurs in the separation membrane module 2B. The precipitates on the membrane are removed by cross flow fermentation liquid in the separation membrane module 2B.

After a certain period of time (for example, one minute later), the filtration-stop treatment by the separation membrane module 2B is terminated and filtration treatment is carried out using all of the separation membrane modules 2A, 2B, and 2C (Step S5). By switching the filtration valve 15B to an open state and driving the filtration pump 11B, the filtration treatment is performed in all of the separation membrane modules 2A, 2B, and 2C.

After a certain period of time (for example, two minutes later), the filtration-stop treatment by the separation membrane module 2C is performed (Step S6). When the filtration-stop treatment is performed with the separation membrane module 2C and remaining separation membrane modules 2A and 2B are used for filtration treatment, by switching the filtration valve 15C to a closed state and stopping the filtration pump 11C, filtration occurs only in the separation membrane modules 2A and 2B, and the filtration-stop treatment occurs in the separation membrane module 2C. The precipitates on the membrane are removed by cross flow fermentation liquid in the separation membrane module 2C.

By repeating the intermittent filtration treatment described above, control can be made such that the filtration-stop treatments of the separation membrane modules 2A, 2B, and 2C are not overlapped with each other.

According to the flow described above, the fermentation liquid is also passed through the separation membrane module 2 having filtration-stop by means of the circulation pump 8. However, it is possible to stop the pass-through of liquid by means of the circulation pump 8 by closing the valve 19 of the separation membrane module 2 with filtration-stop. Since it is expected to remove fouling on the membrane surface of the separation membrane module 2 by shear force generated by cross flow motion, which is caused by the circulation pump 8, it is preferable to have cross flow motion even during filtration-stop.

During the filtration-stop treatment, backwashing of the membrane can be also carried out.

When backwashing is carried out during filtration-stop, during nine minutes of filtration, feedstock is added to a fermentation tank in the same amount as the fermentation liquid reduced by filtration, but during one minute of backwashing during filtration-stop, washing liquid for backwashing flows into the fermentation tank 1, yielding an increased amount of fermentation liquid in the fermentation tank 1. When liquid amount in the fermentation tank 1 is more than the pre-determined value, no feedstock is added to the fermentation tank 1 until the increased amount of backwashing liquid is resolved. When filtration for nine minutes and filtration-stop and backwashing for one minute are repeated all at the same timing with plural separation membrane modules 2, the feedstock is intermittently added so that concentration of feedstock is not stable in the fermentation tank 1 and, as a result, it may be difficult to have stable fermentation. For such reasons, it is effective to have staggered timing of the separation membrane modules 2A, 2B, and 2C so as not to have simultaneous backwashing, and to control to have even filtering amount.

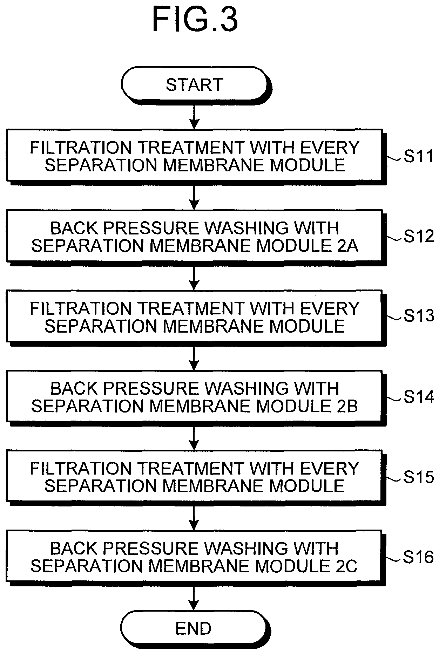

The intermittent filtration treatment in which backwashing is performed during the filtration-stop treatment will be described in view of FIG. 3. FIG. 3 is a flowchart for describing intermittent filtration treatment according to a modification example. When backwashing is performed during filtration-stop of plural separation membrane modules 2A, 2B, and 2C, it is preferable to perform intermittent filtration treatment by controlling the timing of backwashing with the separation membrane modules 2A, 2B, and 2C. Controlling the timing of backwashing means that, backwashing treatment of at least one of the separation membrane modules 2 is performed during the filtration treatment of other separation membrane modules 2, and preferably, backwashing treatments of the separation membrane modules 2A, 2B, and 2C are not overlapped with each other. When an intermittent filtration is performed such that backwashing treatments of the separation membrane modules 2A, 2B, and 2C are not overlapped with each other, the filtration treatment is performed first with all of the separation membrane modules 2A, 2B, and 2C (Step S11). To perform the filtration treatment with all of the separation membrane modules 2A, 2B, and 2C, the filtration valves 15A, 15B, and 15C are open, and the filtration pumps 11A, 11B, and 11C are driven and also fermentation liquid is supplied to the separation membrane modules 2A, 2B, and 2C by means of the circulation pump 8 for filtration. In addition, in all steps described below, the circulating valve 28, the valve 19A, the valve 19B, and the valve 19C, the valve 22A, the valve 22B, and the valve 22C are all in an open state, while the fermentation liquid not filtered by the separation membrane modules 2A, 2B, and 2C is subjected to cross flow to the fermentation tank 1.

After a certain period of time (for example, two minutes later), the separation membrane module 2A is driven for the backwashing treatment (Step S12). When the separation membrane module 2A is driven for the backwashing treatment and remaining separation membrane modules 2B and 2C are driven for filtration treatment, the filtration valves 15B and 15C are open, the filtration valve 15A is closed, the filtration pumps 11B and 11C are driven and the filtration pump 11A is stopped and also fermentation liquid is supplied to the separation membrane modules 2A, 2B, and 2C by means of the circulation pump 8. Further, when the washing liquid valves 16B and 16C are closed, the washing liquid valve 16A is open, the supply pumps 12B and 12C are stopped, and the supply pump 12A is driven, the separation membrane modules 2B and 2C work for filtration and the separation membrane module 2A works for backwashing. In the separation membrane module 2A, washing liquid is supplied to the secondary side of the separation membrane module 2A by means of the supply pump 12A and the washing liquid is filtered at the primary side so as to remove precipitates on the membrane.

After a certain period of time (for example, one minute later), backwashing treatment by the separation membrane module 2A is terminated and the filtration treatment is performed with all of the separation membrane modules 2A, 2B, and 2C (Step S13). Then, by switching the washing liquid valve 16A to a closed state, stopping the supply pump 12A, switching the filtration valve 15A to an open state, and driving the filtration pump 11A, the filtration treatment is carried out with all of the separation membrane modules 2A, 2B, and 2C.

After a certain period of time (for example, two minutes later), the separation membrane module 2B is driven for the backwashing treatment (Step S14). When the separation membrane module 2B is driven for the backwashing treatment and remaining separation membrane modules 2A and 2C are driven for filtration treatment, by switching the filtration valve 15B to a closed state, stopping the filtration pump 11B, switching the washing liquid valve 16B to an open state, and driving the supply pump 12B, the backwashing treatment is carried out with the separation membrane module 2B while the filtration occurs in the separation membrane modules 2A and 2C. In the separation membrane module 2B, washing liquid is supplied to the secondary side of the separation membrane module 2B by means of the supply pump 12B and the washing liquid is filtered at the primary side so as to remove precipitates on the membrane.

After a certain period of time (for example, one minute later), backwashing treatment by the separation membrane module 2B is terminated and the filtration treatment is performed with all of the separation membrane modules 2A, 2B, and 2C (Step S15). Then, by switching the washing liquid valve 16B to a closed state, stopping the supply pump 12B, switching the filtration valve 15B to an open state, and driving the filtration pump 11B, the filtration treatment is carried out with all of the separation membrane modules 2A, 2B, and 2C.

After a certain period of time (for example, two minutes later), the separation membrane module 2C is driven for the backwashing treatment (Step S16). When the separation membrane module 2C is driven for the backwashing treatment and remaining separation membrane modules 2A and 2B are driven for filtration treatment, by switching the filtration valve 15C to a closed state, stopping the filtration pump 11C, switching the washing liquid valve 16C to an open state, and driving the supply pump 12C, the backwashing treatment is carried out with the separation membrane module 2C while the filtration occurs in the separation membrane modules 2A and 2B. In the separation membrane module 2C, washing liquid is supplied to the secondary side of the separation membrane module 2C by means of the supply pump 12C and the washing liquid is filtered at the primary side so as to remove precipitates on the membrane.

By repeating the intermittent filtration treatment described above, control can be made such that the backwashing treatments of the separation membrane modules 2A, 2B, and 2C are not overlapped with each other.

In the above, explanations are given for a case in which backwashing of a membrane is performed during the filtration-stop treatment of the intermittent filtration treatment. However, it is not necessary to perform backwashing during every filtration-stop treatment of the intermittent filtration treatment. Should clogging of separation membrane be prevented, backwashing can be performed for only part of the filtration-stop treatment. For example, it is possible to repeat alternately an intermittent filtration treatment 1 (control is made such that filtration-stop treatments of the separation membrane modules 2A, 2B, and 2C are not overlapped) illustrated in FIG. 2 and an intermittent filtration treatment 2 (control is made such that backwashing treatments of the separation membrane modules 2A, 2B, and 2C are not overlapped) illustrated in FIG. 3. It is also possible to repeat a process including performing the intermittent filtration treatment 1 twice in a row followed by performing the intermittent filtration treatment 2 once, and it can be determined in consideration of performance of a separation membrane module and filtration conditions such as a subject for filtration treatment or the amount of filtration treatment. Alternatively, it is also possible that back liquid washing is not performed during the filtration-stop treatment of intermittent filtration treatment but performed during a separate step instead so that the backwashing step of the separation membrane module 2 can be controlled not to be overlapped with each other.

Timing of the filtration-stop treatment or backwashing treatment in the separation membrane modules 2A, 2B, and 2C is controlled to have a staggered manner, and thus fluctuation in fermentation liquid amount or fluctuation in a supply amount of culture medium is decreased, enabling stable fermentation and recovery of a chemical with high recovery rate.

In the membrane separation type continuous fermentation apparatus 100 used in the first embodiment, three separation membrane modules 2A, 2B, and 2C are arranged in parallel, thus operation can be made in three series. However, the number of the separation membrane modules 2 is not particularly limited, if it is two or more. For example, even for a case in which a membrane separation step is performed by arranging several tens of the separation membrane modules 2 in parallel, filtration-stop treatment (backwashing treatment) of at least one of the separation membrane modules 2 can be performed during the filtration treatment of other separation membrane modules.

Further, for a case in which the separation membrane module 2 is used in great number, timing of the filtration-stop treatment or backwashing treatment of the respective separation membrane modules 2 may be controlled such that the amount of non-filtered liquid returned to the fermentation tank 1 or a fluctuation amount of washing liquid per time is kept at low level. In order to keep the amount of non-filtered liquid returned to the fermentation tank 1 or a fluctuation amount of washing liquid per time at low level, the number of the separation membrane modules 2 for performing the filtration-stop treatment or backwashing treatment is evenly dispersed to perform an intermittent filtration treatment. For example, when an intermittent filtration treatment which includes repeating filtration for nine minutes and filtration-stop for one minute by using 20 separation membrane modules 2 that are arranged in parallel is performed, fluctuation in fermentation liquid amount or a supply amount of culture medium can be kept at low level by performing in order a pair of filtration-stop treatment for one minute, and as a result, stable fermentation can be achieved. It is also the same for performing backwashing during the filtration-stop treatment for one minute, and as a result, not only the fluctuation in fermentation liquid amount or a supply amount of culture medium can be kept at low level but also fluctuation in pH of fermentation liquid can be kept at low level.

In addition, when an intermittent filtration treatment which includes repeating filtration for nine minutes and filtration-stop for one minute by using 12 separation membrane modules 2 that are arranged in parallel is performed, for one intermittent treatment step (i.e., for 10 minutes), it is not possible to control such that the filtration-stop treatments are not overlapped for all 12 separation membrane modules 2. In addition, for one intermittent treatment step, it is not possible to control such that the filtration-stop treatment is evenly performed for all of the separation membrane modules 2. Thus, for such case, it may be considered that a pair of the filtration-stop treatment for one minute is performed in order and the filtration-stop treatment is performed evenly for all of the separation membrane modules 2 during six intermittent filtration treatments (e.g., one minute × five times for all of the separation membrane modules 2).

It is preferable that the fermentation liquid be evenly transported to each separation membrane module 2. Thus, depending on viscosity of fermentation liquid to be transported and length and thickness of the pipe of the liquid transporting line, small liquid transporting resistance per liquid transporting pressure is preferable. The number of the series is preferably determined in view of the specifications of the circulation pump 8 in addition to driving mode of the separation membrane module 2.

(First Embodiment)

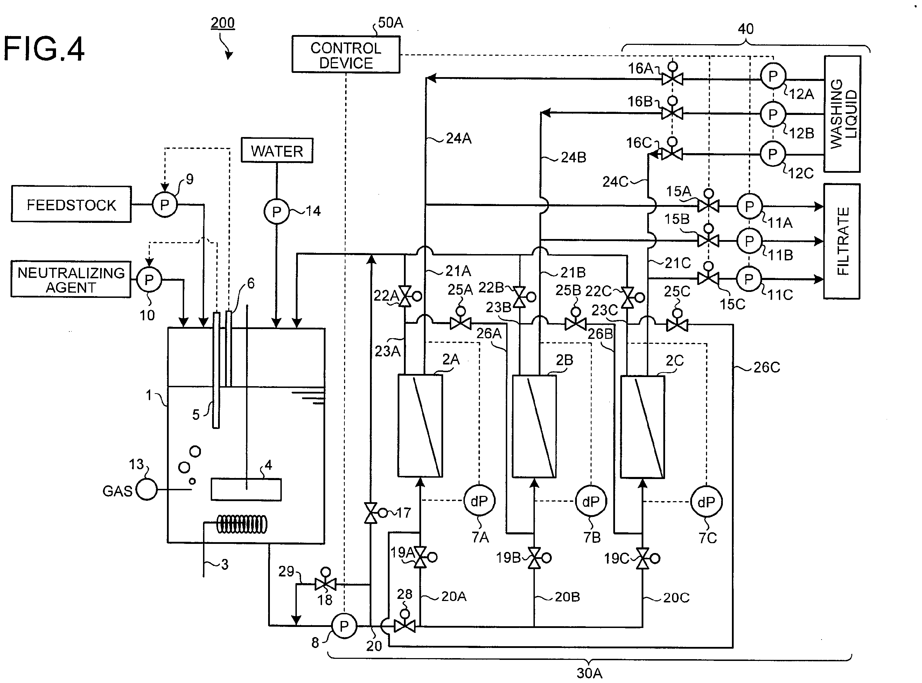

FIG. 4 is a schematic diagram illustrating a membrane separation type continuous fermentation apparatus that is used in a first embodiment of the invention. A membrane separation type continuous fermentation apparatus 200 is different from the membrane separation type continuous fermentation apparatus 100 in that it is equipped with pipes 26A, 26B, and 26C for arranging three separation membrane modules 2A, 2B, and 2C in series. Hereinafter, the membrane separation type continuous fermentation apparatus 200 will be described.

A separation membrane unit 30A has the pipe 26A, the pipe 26B, and the pipe 26C which are branched from the pipe 23A, the pipe 23B, and the pipe 23C, respectively. On the pipe 26A, the pipe 26B, and the pipe 26C, valves 25A, 25B, and 25C are placed, respectively. The pipe 26A connects the primary side of the separation membrane module 2A to the primary side of the separation membrane module 2B, the pipe 26B connects the primary side of the separation membrane module 2B to the primary side of the separation membrane module 2C, and the pipe 26C connects the primary side of the separation membrane module 2C to the primary side of the separation membrane module 2A.

In the separation membrane unit 30A, by opening the circulating valve 28, the valve 19A, the valves 25A and 25B, and the valve 22C and closing the valves 19B and 19C, the valves 22A and 22B, and the valve 25C, fermentation liquid is transported from the fermentation tank 1 to the separation membrane module 2A by means of the circulation pump 8, and the fermentation liquid not filtered by the separation membrane module 2A is transported to the separation membrane module 2B via the pipe 26A. Further, the fermentation liquid not filtered by the separation membrane module 2B is transported to the separation membrane module 2C via the pipe 26B and the fermentation liquid not filtered by the separation membrane module 2C is returned to the fermentation tank 1 via the pipe 23C. When three separation membrane modules 2A, 2B, and 2C are arranged in series, compared to a case in which they are arranged in parallel, total cross flow rate can be cut to 1/3 while the cross flow flux is maintained.

When the cross flow rate is high, a large-size equipment and facilities such as pipe for transportation of liquid from the fermentation tank 1 to the separation membrane modules 2A, 2B, and 2C, valves, and liquid transporting pumps are needed. As a result, capacity of the liquid transporting pipe or the like is increased. In this regard, although environment for fermentation culture is suitably controlled in the fermentation tank 1 by supply of feedstock or oxygen for aerobic fermentation, capacity of the liquid transporting pipe or the like other than the fermentation tank 1 is relatively large compared to capacity of the fermentation tank 1, and thus the fermentation efficiency may be lowered. There is also a problem of high cost including increased facility cost caused by having large facilities and increased power consumption by a liquid transporting pump due to increased cross flow rate. In the membrane separation type continuous fermentation apparatus 200, by arranging the separation membrane modules 2A, 2B, and 2C in series to lower cross flow rate, the above problem can be solved.

Further, by changing opening and closing of each valve in the separation membrane unit 30A, order of transporting fermentation liquid to the separation membrane modules 2A, 2B, and 2C can be modified. For example, when fermentation liquid is transported first to the separation membrane module 2B, the circulating valve 28, the valve 19B, the valves 25B and 25C and the valve 22A are open, and the valves 19A and 19C, the valves 22B and 22C, and the valve 25A are closed. Accordingly, fermentation liquid is first transported from the fermentation tank 1 to the separation membrane module 2B by means of the circulation pump 8, and the fermentation liquid not filtered by the separation membrane module 2B is transported to the separation membrane module 2C via the pipe 26B. Further, the fermentation liquid not filtered by the separation membrane module 2C is transported to the separation membrane module 2A via the pipe 26C and the fermentation liquid not filtered by the separation membrane module 2A is returned to the fermentation tank 1 via the pipe 23A.

Similarly, when fermentation liquid is transported first to the separation membrane module 2C, the circulating valve 28, the valve 19C, the valves 25A and 25C, and the valve 22B are open, and the valves 19A and 19B, the valves 22A and 22C, and the valve 25B are closed. Accordingly, fermentation liquid is first transported from the fermentation tank 1 to the separation membrane module 2C by means of the circulation pump 8, and the fermentation liquid not filtered by the separation membrane module 2C is transported to the separation membrane module 2A via the pipe 26C. Further, the fermentation liquid not filtered by the separation membrane module 2A is transported to the separation membrane module 2B via the pipe 26A and the fermentation liquid not filtered by the separation membrane module 2B is returned to the fermentation tank 1 via the pipe 23B.

When the separation membrane modules 2A, 2B, and 2C are arranged in series, the fermentation liquid supply pressure at the primary side of the separation membrane of the subsequent separation membrane module is lowered as much as pressure loss in the separation membrane module caused by cross flow. For such reasons, when pressure control at the secondary side of the separation membrane is not performed, the front separation membrane module has larger separation membrane pressure difference than the subsequent separation membrane module, and as a result, when shapes and membrane areas of the front and subsequent separation membrane modules are the same, at initial stage of filtration, filtration rate is faster in the front separation membrane module while clogging occurs also faster in the front separation membrane module compared to the subsequent separation membrane module. When the separation membrane unit 30A according to the first embodiment is driven by changing the order of transporting fermentation liquid to the separation membrane modules 2A, 2B, and 2C, filtration amount by the separation membrane modules 2A, 2B, and 2C is adjusted so that clogging of the separation membranes of the separation membrane modules 2A, 2B, and 2C can be prevented.

Timing for changing the order of transporting fermentation liquid to the separation membrane modules 2A, 2B, and 2C can be appropriately determined. Alternatively, it is also possible to change the order when it is found to have progressed clogging of the separation membranes caused by pressure difference of the separation membranes, which is measured for the separation membrane modules 2A, 2B, and 2C by using pressure difference sensors 7A, 7B, and 7C.

Next, the intermittent filtration treatment by the separation membrane unit 30A according to the first embodiment will be described. The intermittent filtration treatment by the separation membrane unit 30A can be performed with the same steps as the intermittent filtration treatment according to FIG. 2. When the intermittent filtration treatment is performed such that the filtration-stop treatments of the separation membrane modules 2A, 2B, and 2C are not overlapped with each other, the filtration treatment is carried out first with all of the separation membrane modules 2A, 2B, and 2C (Step S1). In order to perform the filtration treatment with all of the separation membrane modules 2A, 2B, and 2C, the filtration valves 15A, 15B, and 15C are open, and with driving the filtration pumps 11A, 11B, and 11C, fermentation liquid is supplied to the separation membrane modules 2A, 2B, and 2C in order by means of the circulation pump 8 to have filtration of fermentation liquid in the separation membrane modules 2A, 2B, and 2C. Further, in every step described below, the valve 19A, the valves 25A and 25B, and the valve 22C are open, the valves 19B and 19C, the valves 22A and 22B, and the valve 25C are closed, the fermentation liquid is transported to the separation membrane modules 2A, 2B, and 2C in order, and the fermentation liquid not filtered by the separation membrane module 2C is returned to the fermentation tank 1.

After a certain period of time (for example, two minutes later), the separation membrane module 2A is driven for the filtration-stop treatment (Step S2). When the separation membrane module 2A is driven for the filtration-stop treatment and remaining separation membrane modules 2B and 2C are driven for filtration treatment, the filtration valve 15B and 15C are open, the filtration valve 15A is closed, the filtration pumps 11B and 11C are driven and the filtration pump 11A is stopped and also fermentation liquid is supplied to the separation membrane modules 2A, 2B, and 2C by means of the circulation pump 8, and as a result, filtration occurs only in the separation membrane modules 2B and 2C. The separation membrane module 2A has the filtration-stop treatment and the precipitates on the membrane are removed by cross flow fermentation liquid in the separation membrane module 2A.

After a certain period of time (for example, one minute later), the filtration-stop treatment by the separation membrane module 2A is terminated and filtration treatment is carried out using all of the separation membrane modules 2A, 2B, and 2C (Step S3). By switching the filtration valve 15A to an open state and driving the filtration pump 11A, the filtration treatment is performed in all of the separation membrane modules 2A, 2B, and 2C.

After a certain period of time (for example, two minutes later), the filtration-stop treatment by the separation membrane module 2B is performed (Step S4). When the filtration-stop treatment is performed with the separation membrane module 2B and remaining separation membrane modules 2A and 2C are used for filtration treatment, by switching the filtration valve 15B to a closed state and stopping the filtration pump 11B, filtration occurs only in the separation membrane modules 2A and 2C, and the filtration-stop treatment occurs in the separation membrane module 2B. The precipitates on the membrane are removed by cross flow fermentation liquid in the separation membrane module 2B.

After a certain period of time (for example, one minute later), the filtration-stop treatment by the separation membrane module 2B is terminated and filtration treatment is carried out using all of the separation membrane modules 2A, 2B, and 2C (Step S5). By switching the filtration valve 15B to an open state and driving the filtration pump 11B, the filtration treatment is performed in all of the separation membrane modules 2A, 2B, and 2C.

After a certain period of time (for example, two minutes later), the filtration-stop treatment by the separation membrane module 2C is performed (Step S6). When the filtration-stop treatment is performed with the separation membrane module 2C and remaining separation membrane modules 2A and 2B are used for filtration treatment, by switching the filtration valve 15C to a closed state and stopping the filtration pump 11C, filtration occurs only in the separation membrane modules 2A and 2B, and the filtration-stop treatment occurs in the separation membrane module 2C. The precipitates on the membrane are removed by cross flow fermentation liquid in the separation membrane module 2C.

By repeating the intermittent filtration treatment described above, control can be made such that the filtration-stop treatments of the separation membrane modules 2A, 2B, and 2C are not overlapped with each other.

It is preferable to perform in order the intermittent filtration treatment described above by changing the order of transporting fermentation liquid to the separation membrane modules 2A, 2B, and 2C based on opening and closing of each valve.

Further, with regard to the separation membrane unit 30A according to the first embodiment, backwashing of the separation membrane can be performed during the filtration-stop treatment of the intermittent filtration treatment. The intermittent filtration treatment including backwashing step of the separation membrane unit 30A can be performed with the same steps as the intermittent filtration treatment of FIG. 3.

When an intermittent filtration is performed such that backwashing treatments of the separation membrane modules 2A, 2B, and 2C are not overlapped with each other, the filtration treatment is performed first with all of the separation membrane modules 2A, 2B, and 2C (Step S11). To perform the filtration treatment with all of the separation membrane modules 2A, 2B, and 2C, the filtration valves 15A, 15B, and 15C are open, the filtration pumps 11A, 11B, and 11C are driven, and also fermentation liquid is supplied to the separation membrane modules 2A, 2B, and 2C in order by means of the circulation pump 8 for filtration in the separation membrane modules 2A, 2B, and 2C. In addition, in all steps described below, the valve 19A, the valves 25A and 25B, and the valve 22C are open, the valves 19B and 19C, the valves 22A and 22B, and the valve 25C are closed, and the fermentation liquid is transported in order to the separation membrane modules 2A, 2B, and 2C while the fermentation liquid not filtered by the separation membrane module 2C is returned to the fermentation tank 1.

After a certain period of time (for example, two minutes later), the separation membrane module 2A is driven for the backwashing treatment (Step S12). When the separation membrane module 2A is driven for the backwashing treatment and remaining separation membrane module 2B and 2C are driven for filtration treatment, the filtration valve 15B and 15C are open, the filtration valve 15A is closed, the filtration pumps 11B and 11C are driven and the filtration pump 11A is stopped and also fermentation liquid is supplied to the separation membrane modules 2A, 2B, and 2C by means of the circulation pump 8. Further, when the washing liquid valve 16B and 16C are closed, the washing liquid valve 16A is open, the supply pumps 12B and 12C are stopped, and the supply pump 12A is driven, and thus the separation membrane module 2B and 2C work for filtration and the separation membrane module 2A works for backwashing. In the separation membrane module 2A, washing liquid is supplied to the secondary side of the separation membrane module 2A by means of the supply pump 12A and the washing liquid is filtered at the primary side so as to remove precipitates on membrane.

After a certain period of time (for example, one minute later), backwashing treatment by the separation membrane module 2A is terminated and the filtration treatment is performed with all of the separation membrane modules 2A, 2B, and 2C (Step S13). Then, by switching the washing liquid valve 16A to a closed state, stopping the supply pump 12A, switching the filtration valve 15A to an open state, and driving the filtration pump 11A, the filtration treatment is carried out with all of the separation membrane modules 2A, 2B, and 2C.

After a certain period of time (for example, two minutes later), the separation membrane module 2B is driven for the backwashing treatment (Step S14). When the separation membrane module 2B is driven for the backwashing treatment and remaining separation membrane modules 2A and 2C are driven for filtration treatment, by switching the filtration valve 15B to a closed state, stopping the filtration pump 11B, switching the washing liquid valve 16B to an open state, and driving the supply pump 12B, the backwashing treatment is carried out with the separation membrane module 2B while filtration is achieved by the separation membrane modules 2A and 2C. In the separation membrane module 2B, washing liquid is supplied to the secondary side of the separation membrane module 2B by means of the supply pump 12B and the washing liquid is filtered at the primary side so as to remove precipitates on membrane.

After a certain period of time (for example, one minute later), backwashing treatment by the separation membrane module 2B is terminated and the filtration treatment is performed with all of the separation membrane modules 2A, 2B, and 2C (Step S15). Then, by switching the washing liquid valve 16B to a closed state, stopping the supply pump 12B, switching the filtration valve 15B to an open state, and driving the filtration pump 11B, the filtration treatment is carried out with all of the separation membrane modules 2A, 2B, and 2C.

After a certain period of time (for example, two minutes later), the separation membrane module 2C is driven for the backwashing treatment (Step S16). When the separation membrane module 2C is driven for the backwashing treatment and remaining separation membrane modules 2A and 2B are driven for filtration treatment, by switching the filtration valve 15C to a closed state, stopping the filtration pump 11C, switching the washing liquid valve 16C to an open state, and driving the supply pump 12C, the backwashing treatment is carried out with the separation membrane module 2C while filtration is achieved by the separation membrane modules 2A and 2B. In the separation membrane module 2C, washing liquid is supplied to the secondary side of the separation membrane module 2C by means of the supply pump 12C and the washing liquid is filtered at the primary side so as to remove precipitates on membrane.

By repeating the intermittent filtration treatment described above, control can be made such that the backwashing treatments of the separation membrane modules 2A, 2B, and 2C are not overlapped with each other.

Further, it is preferable to perform in order the intermittent filtration treatment described above by changing the order of transporting fermentation liquid to the separation membrane modules 2A, 2B, and 2C based on opening and closing of each valve.

In the above, backwashing of membrane during the filtration-stop treatment of intermittent filtration treatment was described. However, it is not necessary to perform backwashing during every filtration-stop treatment of intermittent filtration treatment. Instead, should clogging of separation membrane be prevented, backwashing can be performed for only part of the filtration-stop treatment. Alternatively, it is also possible that back liquid washing is not performed during the filtration-stop treatment of intermittent filtration treatment but performed as a separate step so that control is made such that the backwashing steps of the separation membrane modules 2 are not overlapped.

Further, in the membrane separation type continuous fermentation apparatus 200, culture medium is supplied to the fermentation tank 1 by means of the culture medium supply pump 9. If necessary, it is also possible that the fermentation liquid in the fermentation tank 1 is stirred by means of the stirring device 4 and required gas is supplied by means of the gas supply device 13. Gas may be supplied to the separation membrane modules 2A, 2B, and 2C, and precipitates on surface of separation membrane can be removed by shear force of gas. For aerobic fermentation, in particular, oxygen dissolving efficiency may be increased.

When the separation membrane modules 2A, 2B, and 2C are arranged in series, gas may be supplied from the first module in the series or from a separation membrane module present between the first module and the last module. When gas is supplied to the separation membrane module 2, from the viewpoint of separation membrane washing effect by gas, it is preferable that gas is supplied from the first separation membrane module 2. In such case, the supplied gas may be collected and recycled for supply by means of the gas supply device 13.

According to the first embodiment, control is made to have staggered timing of the filtration-stop treatment or backwashing treatment of the separation membrane modules 2A, 2B, and 2C as described above, which yields less fluctuation in the amount of fermentation liquid or less fluctuation in a supply amount of culture medium. As a result, fermentation can be performed stably and a chemical can be recovered with high recovery ratio. In addition, by arranging the separation membrane modules 2A, 2B, and 2C in series, a total amount of cross flow of fermentation liquid circulating in the apparatus can be reduced, and thus the apparatus size can be minimized and running cost can be lowered.

In the membrane separation type continuous fermentation apparatus 200, three separation membrane modules 2A, 2B, and 2C are arranged in series, thus operation can be made in three series. However, the number of the separation membrane modules 2 is not particularly limited, if it is two or more. For example, even for a case in which a membrane separation step is performed by arranging several tens of separation membrane modules 2 in parallel, at least one filtration-stop treatment (backwashing treatment) of the separation membrane module 2 can be performed during a filtration treatment of other separation membrane module.

Further, for a case in which the separation membrane module 2 is used in great number, timing of the filtration-stop treatment or backwashing treatment may be controlled such that the amount of non-filtered liquid returned to the fermentation tank 1 or a fluctuation amount of washing liquid per time is kept at low level. In order to keep the amount of non-filtered liquid returned to the fermentation tank 1 or a fluctuation amount of washing liquid per time at low level, control may be made such that the number of the separation membrane modules 2 for performing the filtration-stop treatment or backwashing treatment is evenly dispersed to have an intermittent filtration treatment.

In the first embodiment, it is preferable that the fermentation liquid is evenly filtered in each separation membrane module 2 by controlling the order of liquid transport or transmembrane pressure. The number of the separation membrane modules 2 that are installed is preferably determined in view of the specifications of the used circulation pump 8 in addition to driving mode of the separation membrane module 2.

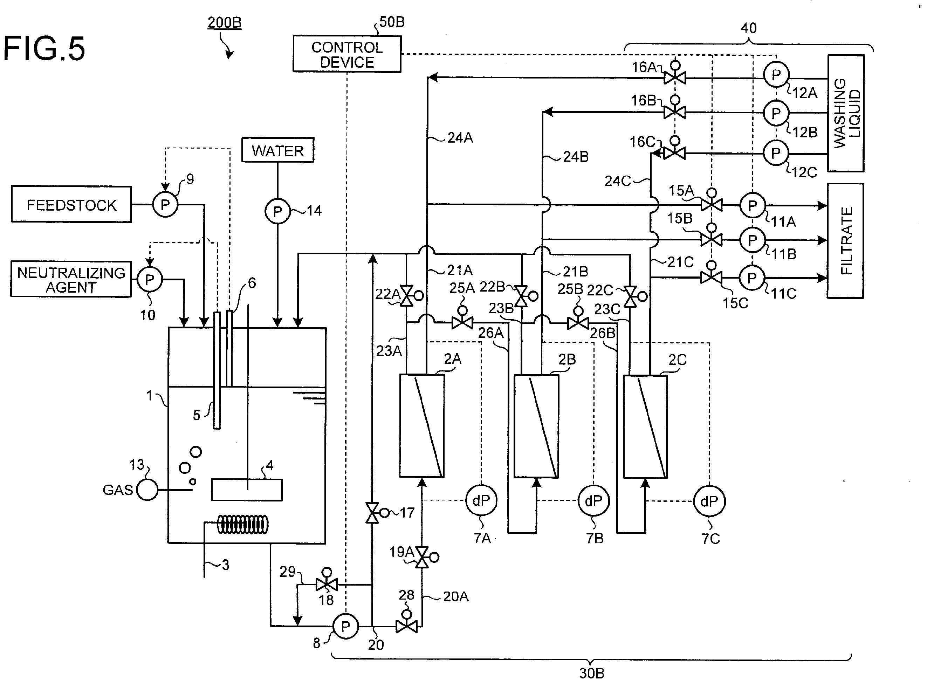

Further, as the membrane separation type continuous fermentation apparatus having the separation membrane modules 2A, 2B, and 2C arranged in series, the apparatus illustrated in FIG. 5 can be used. FIG. 5 is a schematic diagram illustrating a membrane separation type continuous fermentation apparatus. In a membrane separation type continuous fermentation apparatus 200A, each module is arranged in series so that fermentation liquid is transported in order of the separation membrane modules 2A, 2B, and 2C by means of the circulation pump 8. In the membrane separation type continuous fermentation apparatus 200A, pipes or or the like are not present for changing the liquid transport order in the separation membrane modules 2A, 2B, and 2C, and therefore the liquid transport order cannot be changed.

According to the membrane separation type continuous fermentation apparatus, the fermentation liquid transport order is fixed. Thus, the separation membrane of the front separation membrane module 2A may be easily clogged. In order to prevent clogging of the separation membrane of the separation membrane module 2A, pressure differences of separation membranes of the separation membrane modules 2A, 2B, and 2C are measured by means of the pressure difference sensors 7A, 7B, and 7C, and it is preferable to control to have approximately constant pressure difference of each separation membrane. In order to control to have approximately constant pressure difference of the separation membrane of the separation membrane modules 2A, 2B, and 2C, opening level of the valve 15A, 15B, or 15C can be modified, i.e., the opening level of valve can be controlled either automatically or manually to have constant filtration flow rate per separation membrane module.

According to the membrane separation type continuous fermentation apparatus 200A, there is no unused liquid transporting line. Thus, death of microorganisms caused by retained fermentation liquid to yield materials for causing contaminant of separation membrane and lowering the filtration performance can be prevented.

(Second Embodiment)

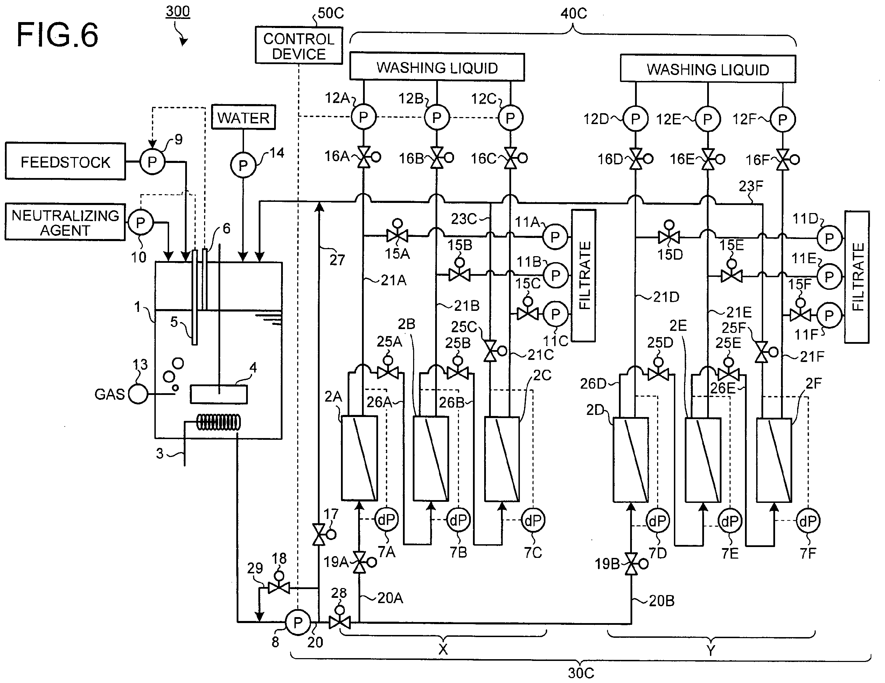

FIG. 6 is a schematic diagram illustrating a membrane separation type continuous fermentation apparatus that can be used for a second embodiment of the invention. A membrane separation type continuous fermentation apparatus 300 includes the separation membrane line X in which three separation membrane modules 2A, 2B, and 2C are arranged in series and the separation membrane line Y in which three separation membrane modules 2D, 2E, and 2F are arranged in series, in which the separation membrane line X and the separation membrane line Y are arranged in parallel.

The separation membrane line X consists of three separation membrane modules 2A, 2B, and 2C, and the fermentation liquid supplied via the pipe 20A by means of the circulation pump 8 is first transported to the separation membrane module 2A. The fermentation liquid filtered by the separation membrane module 2A is discharged to outside of the apparatus via the pipe 21A. The fermentation liquid not filtered by the separation membrane module 2A is transported to the separation membrane module 2B via the pipe 26A. The fermentation liquid supplied to the separation membrane module 2B and filtered therein is discharged to outside of the apparatus via the pipe 21B, and non-filtered fermentation liquid is transported to the separation membrane module 2C via the pipe 26B. The fermentation liquid supplied to the separation membrane module 2C and filtered therein is discharged to outside of the apparatus via the pipe 21C, and non-filtered fermentation liquid is returned to the fermentation tank 1 via the pipe 23C.

The separation membrane line Y consists of three separation membrane modules 2D, 2E, and 2F, and the fermentation liquid supplied via the pipe 20B by means of the circulation pump 8 is first transported to the separation membrane module 2D. The fermentation liquid filtered by the separation membrane module 2D is discharged to outside of the apparatus via a pipe 21D. The fermentation liquid not filtered by the separation membrane module 2D is transported to the separation membrane module 2E via a pipe 26D. The fermentation liquid supplied to the separation membrane module 2E and filtered therein is discharged to outside of the apparatus via a pipe 21E, and non-filtered fermentation liquid is transported to the separation membrane module 2F via a pipe 26E. The fermentation liquid supplied to the separation membrane module 2F and filtered therein is discharged to outside of the apparatus via a pipe 21F, and non-filtered fermentation liquid is returned to the fermentation tank 1 via a pipe 23F.

In the separation membrane line X and the separation membrane line Y, in response to pressure decrease of fermentation liquid supplied to a back-end separation membrane module 2 in serial arrangement, it is possible to have the same filtration amount by the separation membrane module 2 in serial arrangement by performing pressure control at secondary side of the separation membrane with an aid of the pressure difference sensor 7, the filtration pump 11, and the filtration valve 15. If pressure control at secondary side of the separation membrane module 2 in serial arrangement is not performed, it is preferable that the number of the separation membrane modules 2 in serial arrangement is limited to a certain number or less.

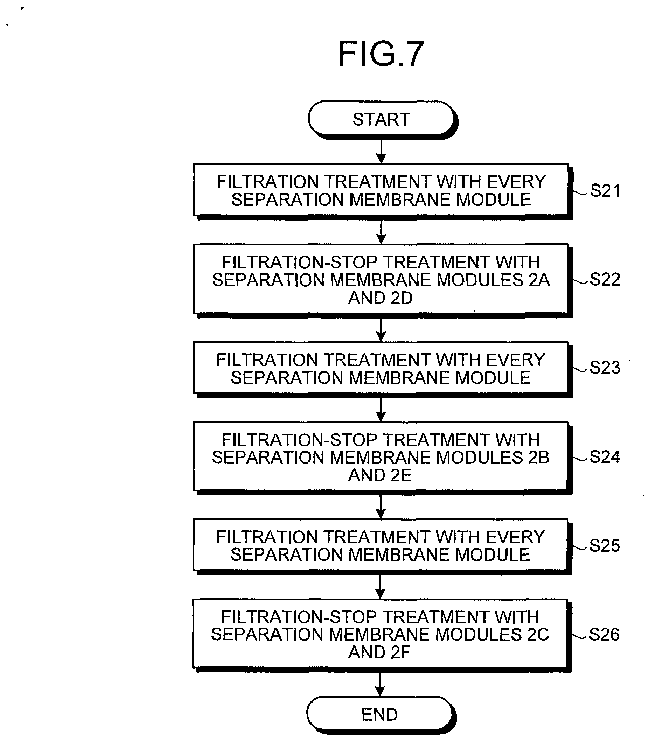

Next, intermittent filtration treatment by a separation membrane unit 30C is described in view of FIG. 7. FIG. 7 is a flow chart for describing intermittent filtration treatment according to the second embodiment. When intermittent filtration is performed using the separation membrane modules 2A to 2F without having an overlapped filtration-stop treatment, the filtration treatment is performed first by using all of the separation membrane modules 2A to 2F (Step S21).

After a certain period of time (for example, two minutes later), the filtration-stop treatment is performed with the separation membrane modules 2A and 2D (Step S22). When the filtration-stop treatment is performed with the separation membrane modules 2A and 2D and filtration treatment is performed with remaining separation membrane modules 2B, 2C, 2E, and 2F, the filtration valve 15A and a filtration valve 15D are closed and the filtration pump 11A and a filtration pump 11D are stopped.

After a certain period of time (for example, one minute later), the filtration-stop treatment by the separation membrane modules 2A and 2D is terminated and filtration treatment is performed with all of the separation membrane modules 2A to 2F (Step S23).

After a certain period of time (for example, two minutes later), the filtration-stop treatment is performed with the separation membrane modules 2B and 2E (Step S24). When the filtration-stop treatment is performed with the separation membrane modules 2B and 2E and filtration treatment is performed with remaining separation membrane modules 2A, 2C, 2D and 2F, the filtration valve 15E and a filtration valve 15E are closed and the filtration pump 11B and a filtration pump 11E are stopped.

After a certain period of time (for example, one minute later), the filtration-stop treatment by the separation membrane modules 2B and 2E is terminated and filtration treatment is performed with all of the separation membrane modules 2A to 2F (Step S25).

After a certain period of time (for example, two minutes later), the filtration-stop treatment is performed with the separation membrane modules 2C and 2F (Step S26). When the filtration-stop treatment is performed with the separation membrane modules 2C and 2F and filtration treatment is performed with remaining separation membrane modules 2A, 2B, 2D and 2E, the filtration valve 15C and a filtration valve 15F are closed and the filtration pump 11C and a filtration pump 11F are stopped.

By repeating the intermittent filtration treatment described above, control can be made such that the filtration-stop treatments of the separation membrane modules 2A to 2F are not overlapped with each other.

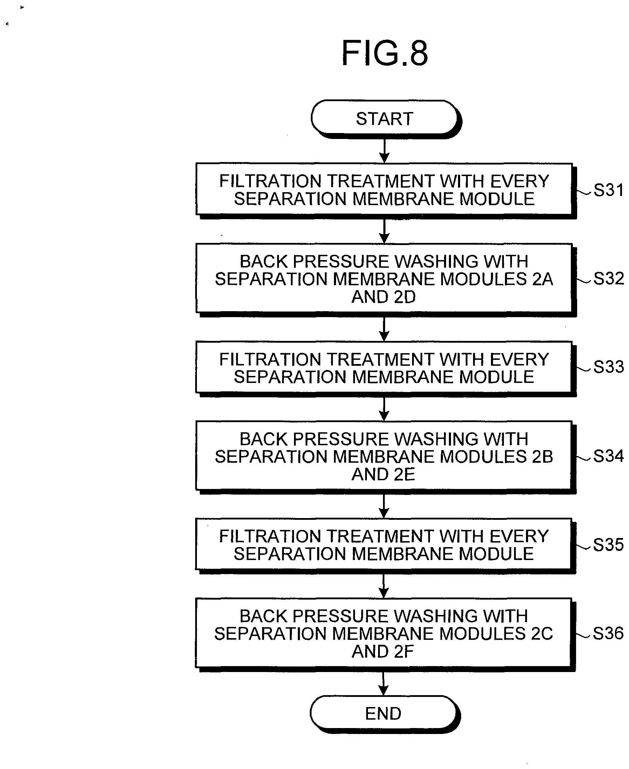

Further, in the separation membrane unit 30C according to the third embodiment, backwashing of membrane can be performed during the filtration-stop treatment. FIG. 8 is a flow chart for describing intermittent filtration treatment including backwashing during filtration-stop treatment in the separation membrane unit C according to the second embodiment of the invention.

When intermittent filtration is performed using the separation membrane modules 2A to 2F without having an overlapped backwashing treatment, filtration treatment is performed first by using all of the separation membrane modules 2A to 2F (Step S31).

After a certain period of time (for example, two minutes later), the backwashing treatment is performed with the separation membrane modules 2A and 2D (Step S32). When the backwashing treatment is performed with the separation membrane modules 2A and 2D and filtration treatment is performed with remaining separation membrane modules 2B, 2C, 2E, and 2F, the filtration valves 15A and 15D are closed and the filtration pumps 11A and 11D are stopped while opening the washing liquid valve 16A and a washing liquid valve 16D and driving the supply pump 12A and a supply pump 12D.

After a certain period of time (for example, one minute later), the backwashing treatment by the separation membrane modules 2A and 2D is terminated and filtration treatment is performed with all of the separation membrane modules 2A to 2F (Step S33).

After a certain period of time (for example, two minutes later), the backwashing treatment is performed with the separation membrane modules 2B and 2E (Step S34). When the backwashing treatment is performed with the separation membrane modules 2B and 2E and filtration treatment is performed with remaining separation membrane modules 2A, 2C, 2D and 2F, the filtration valves 15B and 15E are closed and the filtration pumps 11B and 11E are stopped while opening the washing liquid valve 16B and a washing liquid valve 16E and driving the supply pump 12B and a supply pump 12E.

After a certain period of time (for example, one minute later), the backwashing treatment by the separation membrane modules 2B and 2E is terminated and filtration treatment is performed with all of the separation membrane modules 2A to 2F (Step S35).

After a certain period of time (for example, two minutes later), the backwashing treatment is performed with the separation membrane modules 2C and 2F (Step S36). When the backwashing treatment is performed with the separation membrane modules 2C and 2F and filtration treatment is performed with remaining separation membrane modules 2A, 2B, 2D and 2E, the filtration valves 15C and 15F are closed and the filtration pumps 11C and 11F are stopped while opening the washing liquid valve 16C and a washing liquid valve 16F and driving the supply pump 12C and a supply pump 12F.

By repeating the intermittent filtration treatment described above, control can be made such that backwashing treatments of the separation membrane modules 2A to 2F are not overlapped with each other.

According to the second embodiment, control is made to have staggered timing of the filtration-stop treatment or backwashing treatment of the separation membrane modules 2A to 2F, which yields less fluctuation in the amount of fermentation liquid or less fluctuation in a supply amount of culture medium. As a result, fermentation can be performed stably and a chemical can be recovered with high recovery ratio. In addition, by arranging in multiple rows the separation membrane lines in which plural separation membrane module 2 are arranged in series, clogging of separation membrane is prevented, and as a result, not only a chemical can be recovered stably but also the apparatus size can be minimized and running cost can be lowered.

In the second embodiment, explanations are given for a case in which one of the plural separation membrane modules 2 constituting the separation membrane lines in two parallel rows is used for the filtration-stop treatment. However, it is also possible to control the timing of the filtration-stop treatment of all of the separation membrane modules 2 to have no overlap. For example, even for a case in which a membrane separation step is performed by arranging several tens of separation membrane modules 2 in parallel, at least one filtration-stop treatment (backwashing treatment) of the separation membrane module 2 can be performed during a filtration treatment of other separation membrane module.

Examples

Herein below, the effect of the invention is described in more detail in view of the Examples in which D-lactic acid is selected as a chemical to be produced.

(Reference example 1) Preparation of hollow fiber membrane

A vinylidene fluoride homopolymer having a mass average molecular weight of 417,000 and γ-butyrolactone were melted at a temperature of 170°C in amounts of 38% by mass and 62% by mass, respectively. The resulting polymer solution, accompanied by γ-butyrolactone as a hollow-forming liquid, was discharged from a base and solidified in a cooling bath consisting of 80% by mass of aqueous γ-butyrolactone solution at a temperature of 20°C to prepare a hollow fiber membrane with spherical structure. Then, 14% by mass of vinylidene fluoride homopolymer having a weight-average molecular weight of 284,000, 1% by mass of cellulose acetate propionate (CAP482-0.5, manufactured by Eastman Chemical), 77% by mass of N-methyl-2-pyrrolidone, 5% by mass of T-20C, and 3% by mass of water were mixed and melted at a temperature of 95°C to prepare a polymer solution. The resulting stock solution for forming membrane was applied uniformly onto the surface of the hollow fiber membrane with spherical structure and immediately solidified in a water bath to prepare a hollow fiber membrane in which a three dimensional mesh structure is formed on the spherical structure layer. The resulting hollow fiber membrane had an average pore size of 0.04 $\mu$m on the surface of the water-treated side. When the hollow fiber porous membrane as a separation membrane was evaluated for its purified-water permeability amount, the purified-water permeability amount was found to be 5.5 $\times$ 10$^{-9}$ m$^3$/m$^2$/s/Pa. Measurement of the water permeability amount was conducted with reverse osmosis membrane-treated purified water at 25°C with a head height of 1 m.

(Example 1) (falling outside the scope of the invention)

A separation membrane module was produced by using the hollow fiber membrane of the Reference example 1. As the separation membrane module case, a molded product which is a cylindrical vessel made of polysulfone resin was used for producing a hollow fiber membrane module. By using the porous hollow fiber membrane and membrane filtration module produced above, the Example 1 was carried out. Unless otherwise noted, the operation conditions in the Example 1 are as follows.

Fermentation tank capacity: 2 (L)
Effective volume of fermentation tank: 1.5 (L)
Used separation membrane: 22 polyvinylidene fluoride hollow fiber membranes (effective length: 8 cm and total effective
membrane area: 0.023 (m$^2$))
Number of hollow fiber membrane modules: three, arranged in parallel to have three series
Temperature control: 37 (°C)
Fermentation tank aeration amount: Nitrogen gas of 0.2 (L/min)
Fermentation tank agitation rate: 600 (rpm)
pH adjustment: adjusted to pH 6 by using 3 N Ca(OH)$_2$
Lactic acid fermentation culture medium feed: added to have a constant fermentation tank liquid amount of about 1.5 L
Cross flow flux by fermentation liquid circulating device: 0.3 (m/s)
Flow rate control for membrane filtration: flow rate control by suction pump
Intermittent filtration treatment: periodic operation including filtration treatment (for nine minutes) and filtration-stop treatment (for one minute)
Membrane filtration flux: varied to have transmembrane pressure difference of 20 kPa or less within the range of 0.01 (m/day) to 0.3 (m/day). When transmembrane pressure difference continuously increases over the range, continuous fermentation was terminated.

Culture medium was steam-sterilized for 20 minutes under saturated vapor at 121°C. Sporolactobacillus laevolacticus JCM2513 (SL strain) was used as microorganisms. The lactic acid fermentation culture medium having the composition listed in the Table 1 was used as the culture medium, and the concentration of lactic acid as a product was evaluated by HPLC listed below according to the following conditions.

Table 1
Lactic acid fermentation culture medium

| Components | Amount |
|---|---|
| Glucose | 100 g |
| Yeast Nitrogen base W/O amino acid (Difco Corporation) | 6.7 g |
| Standard 19 kinds of amino acids except leucine | 152 mg |
| Leucine | 760 mg |
| Inositol | 152 mg |
| p-Aminobenzoic acid | 16 mg |
| Adenine | 40 mg |
| Uracil | 152 mg |
| Water | 892 mg |

Column: Shim-Pack SPR-H (trade name, manufactured by SHIMADZU CORPORATION)

Mobile phase: 5 mM p-toluene sulfonic acid (0.8 mL/min)

Reacting phase: 5 mM p-toluene sulfonic acid, 20 mM bistris, 0.1 mM EDTA·2Na (0.8 mL/min)

Detection method: electric conductivity

Column temperature: 45°C

Further, analysis of optical purity of lactic acid (i.e., excess ratio of enantiomers) was performed according to the following conditions.

Column: TSK-gel Enantio L1 (trade name, manufactured by TOSOH CORPORATION)
Mobile phase: 1 mM aqueous solution of copper sulfate
Flux: 1.0 mL/min
Detection method: UV 254 nm
Temperature: 30°C

The optical purity of L-lactic acid is calculated using the following equation (5).

$$\text{Optical purity (\%)} = 100 \times (L - D)/(D + L) \qquad (5)$$

The optical purity of D-lactic acid is calculated using the following equation (6).

$$\text{Optical purity (\%)} = 100 \times (D - L)/(D + L) \qquad (6)$$

Herein, L represents the concentration of L-lactic acid, and D represents the concentration of D-lactic acid.

First, the SL strain was shake-cultured overnight in 5 ml of a lactic acid fermentation culture medium in a test tube (prior preliminary preculture). The resulting culture liquid was inoculated into 100 mL of a fresh lactic acid fermentation culture medium and shake-cultured for 24 hours at 30°C in a 500 mL Sakaguchi flask (preliminary preculture). The preliminary preculture was inoculated into a lactic acid fermentation culture medium in 1.5 L of a fermentation tank in the membrane separation type continuous fermentation apparatus 100 illustrated in FIG. 1, the fermentation tank 1 was stirred by means of the stirring device 4 attached thereto, followed by the aeration control, temperature control, and pH adjustment of the fermentation tank 1, and without operating the circulation pump 8 for fermentation culture liquid, the microorganisms were cultured for 24 hours (preculture). Immediately after preculture was finished, the circulation pump 8 for fermentation culture liquid was operated, and the microorganisms were continuously cultured under the conditions where in addition to the operation conditions at the time of preculture, a lactic acid fermentation culture medium was continuously fed and the amount of membrane permeation water was controlled such that the amount of the fermentation liquid in the continuous fermentation apparatus became 1.5 L, whereby D-lactic acid was produced by continuous fermentation. In this continuous fermentation test, the amount of membrane permeation water was controlled such that filtration amount by the filtration pumps 11A, 11B, and 11C is the same as the flow rate of supplied fermentation culture medium. The concentration of D-lactic acid produced in the membrane permeation fermentation liquid and the residual glucose concentration were measured appropriately.

With regard to the membrane separation type continuous fermentation apparatus 100, an intermittent filtration treatment was performed according to the flow illustrated in FIG. 2 such that the filtration-stop treatments of the separation membrane modules 2A, 2B, and 2C are not overlapped with each other. The intermittent filtration treatment includes a filtration treatment for two minutes using every separation membrane module 2 and a filtration-stop treatment for one minute by using only the separation membrane module 2A. Then, the filtration treatment is performed for two minutes using every separation membrane module 2 and filtration-stop treatment is performed for one minute by using only the separation membrane module 2B. Then, the filtration treatment is performed for two minutes using every separation membrane module 2 and filtration-stop treatment is performed for one minute by using only the separation membrane module 2C. By repeating continuously the intermittent filtration treatment, continuous fermentation was performed and produced D-lactic acid was recovered.

Results of the continuous fermentation test by performing intermittent filtration treatment are summarized in the Table 2. With regard to the membrane separation type continuous fermentation apparatus 100 illustrated in FIG. 1, it was possible to perform continuous fermentation for 380 hours and the maximum production rate of D-lactic acid was 2.8 g/L/hr. In addition, the circulation pump flow rate was 2.5 L/min.

Table 2

| | Example 1 | Example 2 | Comparative example 1 | Example 3 | Example 4 | Comparative example 2 | Example 5 | Example 6 |
|---|---|---|---|---|---|---|---|---|
| Fermentation time [hr] | 380 | 420 | 340 | 370 | 400 | 320 | 400 | 420 |
| Maximum production rate of D-lactic acid [g/L/hr] | 2.8 | 4.0 | 2.4 | 3.8 | 4.2 | 0.7 | 4.5 | 4.4 |

(Example 2) (falling outside the scope of the invention)

In the Example 2, with regard to the membrane separation type continuous fermentation apparatus 100, continuous fermentation of D-lactic acid was preformed while performing backwashing during the filtration-stop treatment of intermittent filtration treatment. The intermittent filtration treatment was performed according to the flow illustrated in FIG. 3 such that the backwashing treatments of the separation membrane modules 2A, 2B, and 2C are not overlapped with each other. The intermittent filtration treatment includes a filtration treatment for two minutes using every separation membrane module 2 and a backwashing treatment for one minute by using only the separation membrane module 2A. Then, the filtration treatment is performed for two minutes using every separation membrane module 2 and backwashing treatment is performed for one minute by using only the separation membrane module 2B. Then, the filtration treatment is performed for two minutes using every separation membrane module 2 and backwashing treatment is performed for one minute by using only the separation membrane module 2C. By repeating continuously the intermittent filtration treatment, continuous fermentation was performed and produced D-lactic acid was recovered. Flux for backwashing was set twice the filtration flux, and the backwashing was performed using distilled water. Other conditions are the same as those of the Example 1.

Results of the continuous fermentation test while performing an intermittent filtration treatment such that the backwashing treatment is performed during the filtration-stop treatment as described above are summarized in the Table 2. With regard to the membrane separation type continuous fermentation apparatus 100 illustrated in FIG. 1, it was possible to perform continuous fermentation for 420 hours and the maximum production rate of D-lactic acid was 4.0 g/L/hr. In addition, the circulation pump flow rate was 2.5 L/min.

(Comparative example 1)

In the Comparative example 1, with the membrane separation type continuous fermentation apparatus 100, continuous fermentation of D-lactic acid was performed while also performing an intermittent filtration treatment including simultaneous filtration-stop treatment of the separation membrane modules 2A, 2B, and 2C. The intermittent filtration treatment includes running every separation membrane module 2 for two minutes followed by running every separation membrane module 2 for one minute for the filtration-stop treatment and running again every separation membrane module 2 for six minutes for filtration. By repeating continuously the intermittent filtration treatment, continuous fermentation was performed and produced D-lactic acid was recovered. Other conditions are the same as those of the Example 1.

Results of the continuous fermentation test by performing an intermittent filtration treatment are summarized in the Table 2. With regard to the membrane separation type continuous fermentation apparatus 100 illustrated in FIG. 1, it was possible to perform continuous fermentation for 340 hours and the maximum production rate of D-lactic acid was 2.4 g/L/hr. In addition, the circulation pump flow rate was 2.5 L/min.

(Example 3)

With regard to the membrane separation type continuous fermentation apparatus 200 illustrated in FIG. 4 in which the separation membrane modules 2A, 2B, and 2C are arranged in series, a continuous fermentation test was conducted while performing an intermittent filtration treatment such that the filtration-stop treatments of the separation membrane modules 2A, 2B, and 2C are not overlapped with each other. Other conditions are the same as those of the Example 1.

With regard to the membrane separation type continuous fermentation apparatus 200, an intermittent filtration treatment was performed according to the flow illustrated in FIG. 2 such that the filtration-stop treatments of the separation membrane modules 2A, 2B, and 2C are not overlapped with each other. The intermittent filtration treatment includes a filtration treatment for two minutes using every separation membrane module 2 and a filtration-stop treatment for one minute by using only the separation membrane module 2A. Then, the filtration treatment is performed for two minutes using every separation membrane module 2 and filtration-stop treatment is performed for one minute by using only the separation membrane module 2B. Then, the filtration treatment is performed for two minutes using every separation membrane module 2 and filtration-stop treatment is performed for one minute by using only the separation membrane module 2C. By repeating continuously the intermittent filtration treatment, continuous fermentation was performed and produced D-lactic acid was recovered.

Results of the continuous fermentation test by performing an intermittent filtration treatment are summarized in the Table 2. With regard to the membrane separation type continuous fermentation apparatus 200 illustrated in FIG. 4, it was possible to perform continuous fermentation for 370 hours and the maximum production rate of D-lactic acid was 3.8 g/L/hr. In addition, the circulation pump flow rate was 0.9 L/min.

(Example 4)

With regard to the membrane separation type continuous fermentation apparatus 200 illustrated in FIG. 4 in which the separation membrane modules 2A, 2B, and 2C are arranged in series, a continuous fermentation test was conducted while performing an intermittent filtration treatment such that a backwashing treatment is performed during the filtration-stop treatment and the backwashing treatments of the separation membrane modules 2A, 2B, and 2C are not overlapped with each other. Flux for backwashing was set twice the filtration flux, and the backwashing was performed using distilled water. Other conditions are the same as those of the Example 1.

With regard to the membrane separation type continuous fermentation apparatus 200, an intermittent filtration treatment was performed according to the flow illustrated in FIG. 3 such that the backwashing treatments of the separation membrane modules 2A, 2B, and 2C are not overlapped with each other. The intermittent filtration treatment includes a filtration treatment for two minutes using every separation membrane module 2 and a backwashing treatment for one minute by using only the separation membrane module 2A. Then, the filtration treatment is performed for two minutes using every separation membrane module 2 and backwashing treatment is performed for one minute by using only the separation membrane module 2B. Then, the filtration treatment is performed for two minutes using every separation membrane module 2 and backwashing treatment is performed for one minute by using only the separation membrane module 2C. By repeating continuously the intermittent filtration treatment, continuous fermentation was performed and produced D-lactic acid was recovered.

Results of the continuous fermentation test by performing an intermittent filtration treatment are summarized in the Table 2. With regard to the membrane separation type continuous fermentation apparatus 200 illustrated in FIG. 4, it was possible to perform continuous fermentation for 400 hours and the maximum production rate of D-lactic acid was 4.2 g/L/hr. In addition, the circulation pump flow rate was 0.9 L/min.

(Comparative example 2)

With regard to the membrane separation type continuous fermentation apparatus 100 illustrated in FIG. 1, the number of hollow fiber membrane modules was adjusted to one (i.e., the separation membrane module 2B and 2C are removed while only the separation membrane module 2A is maintained) and then a continuous fermentation test was performed. The intermittent filtration treatment includes a filtration treatment for two minutes, a filtration-stop treatment for one minute, and a filtration for six minutes. By repeating the intermittent filtration treatment, continuous fermentation was performed and produced D-lactic acid was recovered. Other conditions are the same as those of the Example 1.

Results of the continuous fermentation test by performing an intermittent filtration treatment are summarized in the Table 2. When the chemical was produced using the membrane separation type continuous fermentation apparatus 100 illustrated in FIG. 1, it was possible to perform continuous fermentation for 320 hours and the maximum production rate of D-lactic acid was 0.7 g/L/hr, which is lower than that of the Example 1. In addition, the circulation pump flow rate was 0.9 L/min, because there was only one module.

(Example 5) (falling outside the scope of the invention)

At discharge side of the circulation pump 8 of the membrane separation type continuous fermentation apparatus 100 illustrated in FIG. 1, the circulating valve 28 was installed and fermentation liquid was transported such that fluctuation range of discharge pressure by the circulation pump 8 is 10% and fluctuation cycle is two seconds. Other conditions are the same as those of the Example 2.

Results of the continuous fermentation test are summarized in the Table 2. With regard to the membrane separation type continuous fermentation apparatus 100 illustrated in FIG. 1, it was possible to perform continuous fermentation for 400 hours and the maximum production rate of D-lactic acid was 4.5 g/L/hr. In addition, the circulation pump flow rate was 2.5 L/min.

(Example 6) (falling outside the scope of the invention)

In the Example 6, with regard to the membrane separation type continuous fermentation apparatus 100, continuous fermentation of D-lactic acid was performed while performing backwashing during the filtration-stop treatment of intermittent filtration treatment. The intermittent filtration treatment was controlled according to the flow illustrated in FIG. 3 such that backwashing treatments of the separation membrane modules 2A, 2B, and 2C are not overlapped with each other. The intermittent filtration treatment includes a filtration treatment for two minutes using every separation membrane module 2 and a backwashing treatment for one minute by using only the separation membrane module 2A. Then, the filtration treatment is performed for two minutes using every separation membrane module 2 and backwashing treatment is performed for one minute by using only the separation membrane module 2B. Then, the filtration treatment is performed for two minutes using every separation membrane module 2 and backwashing treatment is performed for one minute by using only the separation membrane module 2C. By repeating continuously the intermittent filtration treatment, continuous fermentation was performed and produced D-lactic acid was recovered. Flux for backwashing was set twice the filtration flux, and the backwashing was performed using 0.005 N aqueous solution of calcium hydroxide. Other conditions are the same as those of the Example 1.

Results of the continuous fermentation test while performing an intermittent filtration treatment such that the backwashing treatment is performed during the filtration-stop treatment as described above are summarized in the Table 2. With regard to the membrane separation type continuous fermentation apparatus 100 illustrated in FIG. 1, it was possible to perform continuous fermentation for 420 hours and the maximum production rate of D-lactic acid was 4.4 g/L/hr. In addition, the circulation pump flow rate was 2.5 L/min.

Industrial Applicability

According to the method of the invention, continuous fermentation enabling maintaining stably the high productivity for a long period of time can be achieved with simple operation condition. Thus, in a general fermentation industry, a chemical as a fermentation product can be produced stably at low cost.

Reference Signs List

1 FERMENTATION TANK
2A, 2B, 2C SEPARATION MEMBRANE MODULE
3 TEMPERATURE CONTROL DEVICE
4 STIRRING DEVICE
5 PH SENSOR·CONTROL DEVICE
6 LEVEL SENSOR·CONTROL DEVICE
7A, 7B, 7C PRESSURE DIFFERENCE SENSOR
8 CIRCULATION PUMP
9 CULTURE MEDIUM SUPPLY PUMP
10 NEUTRALIZING AGENT SUPPLY PUMP
11A, 11B, 11C FILTRATION PUMP
12A, 12B, 12C SUPPLY PUMP
13 GAS SUPPLY DEVICE
14 WATER SUPPLY PUMP
15A, 15B, 15C FILTRATION VALVE
16A, 16B, 16C WASHING LIQUID VALVE
30, 30A, 30B, 30C SEPARATION MEMBRANE UNIT
40, 40C SEPARATION MEMBRANE WASHING DEVICE
50, 50A, 50B, 50C CONTROL DEVICE
100, 200, 200A, 300 MEMBRANE SEPARATION TYPE CONTINUOUS FERMENTATION APPARATUS

**Claims**

1.  A method for producing a chemical by continuous fermentation, comprising:

    a fermentation step for converting a fermentation feedstock, through fermentation by culturing a microorganism or culture cells, into a fermented liquid containing the chemical by a fermentation tank; and
    a membrane separation step for collecting the chemical, as a filtrate, from the fermented liquid with the use of a plurality of separation membrane modules, and returning a non-filtered liquid to a fermented tank,
    wherein in the membrane separation step, an intermittent filtration treatment is performed such that a filtration treatment and a filtration-stop treatment are alternately repeated with the plurality of the separation membrane modules and using the plurality of the separation membrane modules that are arranged in series, and
    the timing of the filtration-stop treatment in each separation membrane module is controlled during the intermittent filtration treatment.

2.  The method for producing a chemical by continuous fermentation according to claim 1,

wherein the filtration-stop treatment of the separation membrane module is controlled such that stopping the filtering operation of at least one separation membrane module is performed during an filtering operation of other separation membrane module.

3. The method for producing a chemical by continuous fermentation according to claim 1 or 2, wherein the filtration-stop treatment of the separation membrane module is controlled such that the filtration-stop treatment of each separation membrane module is not overlapped with each other.

4. The method for producing a chemical by continuous fermentation according to claim 1, wherein in the membrane separation step timing of the filtration-stop treatment of the separation membrane module is controlled to minimize a change in non-filtered liquid amount per hour, which is returned from the separation membrane module to the fermentation tank during the intermittent filtration treatment.

5. The method for producing a chemical by continuous fermentation according to any one of claims 1 to 4, wherein the membrane separation step is performed by backwashing using water as a washing liquid during the filtration-stop treatment.

6. The method for producing a chemical by continuous fermentation according to any one of claims 1 to 4, wherein the membrane separation step is performed during the filtration-stop treatment by backwashing using water containing an oxidizing agent or a reducing agent as a washing liquid.

7. The method for producing a chemical by continuous fermentation according to any one of claims 1 to 4, wherein the membrane separation step is performed during the filtration-stop treatment by backwashing using water containing an acid or an alkali as a washing liquid.

8. The method for producing a chemical by continuous fermentation according to any one of claims 1 to 4, wherein the membrane separation step is performed during the filtration-stop treatment by immersion washing using a washing liquid.

9. The method for producing a chemical by continuous fermentation according to any one of claims 5 to 7, wherein in the membrane separation step timing of the filtration-stop treatment of the separation membrane module is controlled to make an amount of non-filtered liquid which is returned from the separation membrane module to the fermentation tank almost the same as the amount of washing liquid that is used for backwashing.

10. The method for producing a chemical by continuous fermentation according to claim 1, wherein transmembrane pressure is controlled to be constant in each separation membrane module arranged in series.

11. The method for producing a chemical by continuous fermentation according to claim 1 or 10, wherein order of transporting fermentation liquid to a plurality of the separation membrane modules arranged in series can be varied.

12. The method for producing a chemical by continuous fermentation according to any one of claims 1, 10 and 11, wherein the membrane separation step comprises performing, in multiple lines in parallel, an intermittent filtration operation using a separation membrane unit which includes a plurality of the separation membrane modules that are arranged in series.

13. The method for producing a chemical by continuous fermentation according to any one of claims 1 to 12, wherein the membrane separation step comprises performing a filtration treatment by varying pressure of fermentation liquid supplied to primary side of the separation membrane.

**Patentansprüche**

1. Verfahren zur Herstellung einer Chemikalie durch kontinuierliche Fermentation, umfassend:

einen Fermentationsschritt zum Umwandeln eines Fermentationsausgangsmaterials durch Fermentation durch Kultivieren eines Mikroorganismus oder von Kulturzellen in eine fermentierte Flüssigkeit, die die Chemikalie enthält, durch einen Fermentationstank; und

einen Membrantrennschritt zum Sammeln der Chemikalie als Filtrat aus der fermentierten Flüssigkeit unter Verwendung einer Vielzahl von Trennmembranmodulen, und Zurückführen einer ungefilterten Flüssigkeit in einen fermentierten Tank,

wobei im Membrantrennschritt eine intermittierende Filtrationsbehandlung derart durchgeführt wird, dass eine Filtrationsbehandlung und eine Filtrationsstoppbehandlung abwechselnd mit der Vielzahl von Trennmembranmodulen und unter Verwendung der Vielzahl von Trennmembranmodulen, die in Reihe angeordnet sind, wiederholt werden, und

der Zeitpunkt der Filtrationsstoppbehandlung in jedem Trennmembranmodul während der intermittierenden Filtrationsbehandlung kontrolliert wird.

2. Verfahren zur Herstellung einer Chemikalie durch kontinuierliche Fermentation nach Anspruch 1,
wobei die Filtrationsstoppbehandlung des Trennmembranmoduls derart kontrolliert wird, dass das Stoppen des Filtervorgangs mindestens eines Trennmembranmoduls während eines Filtervorgangs eines anderen Trennmembranmoduls durchgeführt wird.

3. Verfahren zur Herstellung einer Chemikalie durch kontinuierliche Fermentation nach Anspruch 1 oder 2,
wobei die Filtrationsstoppbehandlung des Trennmembranmoduls derart kontrolliert wird, dass die Filtrationsstoppbehandlung jedes Trennmembranmoduls nicht miteinander überlappt.

4. Verfahren zur Herstellung einer Chemikalie durch kontinuierliche Fermentation nach Anspruch 1,
wobei in dem Membrantrennschritt der Zeitpunkt der Filtrationsstoppbehandlung des Trennmembranmoduls kontrolliert wird, um eine Änderung der Menge an nicht-filtrierter Flüssigkeit pro Stunde zu minimieren, die während der intermittierenden Filtrationsbehandlung von dem Trennmembranmodul in den Fermentationstank zurückgeführt wird.

5. Verfahren zur Herstellung einer Chemikalie durch kontinuierliche Fermentation nach einem der Ansprüche 1 bis 4,
wobei der Membrantrennschritt durch Rückspülen unter Verwendung von Wasser als Waschflüssigkeit während der Filtrationsstoppbehandlung durchgeführt wird.

6. Verfahren zur Herstellung einer Chemikalie durch kontinuierliche Fermentation nach einem der Ansprüche 1 bis 4,
wobei der Membrantrennschritt während der Filtrationsstoppbehandlung durch Rückspülen unter Verwendung von Wasser, das ein Oxidationsmittel oder ein Reduktionsmittel enthält, als Waschflüssigkeit durchgeführt wird.

7. Verfahren zur Herstellung einer Chemikalie durch kontinuierliche Fermentation nach einem der Ansprüche 1 bis 4,
wobei der Membrantrennschritt während der Filtrationsstoppbehandlung durch Rückspülen unter Verwendung von Wasser, das eine Säure oder ein Alkali enthält, als Waschflüssigkeit durchgeführt wird.

8. Verfahren zur Herstellung einer Chemikalie durch kontinuierliche Fermentation nach einem der Ansprüche 1 bis 4,
wobei der Membrantrennschritt während der Filtrationsstoppbehandlung durch Eintauchwaschen unter Verwendung einer Waschflüssigkeit durchgeführt wird.

9. Verfahren zur Herstellung einer Chemikalie durch kontinuierliche Fermentation nach einem der Ansprüche 5 bis 7,
wobei in dem Membrantrennschritt der Zeitpunkt der Filtrationsstoppbehandlung des Trennmembranmoduls derart kontrolliert wird, dass eine Menge an nicht-filtrierter Flüssigkeit, die von dem Trennmembranmodul in den Fermentationstank zurückgeführt wird, nahezu der Menge an Waschflüssigkeit entspricht, die zum Rückspülen verwendet wird.

10. Verfahren zur Herstellung einer Chemikalie durch kontinuierliche Fermentation nach Anspruch 1,
wobei der Transmembrandruck so kontrolliert wird, dass er in jedem in Reihe angeordneten Trennmembranmodul konstant ist.

11. Verfahren zur Herstellung einer Chemikalie durch kontinuierliche Fermentation nach Anspruch 1 oder 10,
wobei die Reihenfolge des Transports der Fermentationsflüssigkeit zu einer Vielzahl von in Reihe angeordneten Trennmembranmodulen variiert werden kann.

12. Verfahren zur Herstellung einer Chemikalie durch kontinuierliche Fermentation nach einem der Ansprüche 1, 10 und 11,

wobei der Membrantrennschritt das parallele Durchführen eines intermittierenden Filtrationsvorgangs in mehreren Linien unter Verwendung einer Trennmembraneinheit umfasst, die eine Vielzahl von Trennmembranmodulen umfasst, die in Reihe angeordnet sind.

13. Verfahren zur Herstellung einer Chemikalie durch kontinuierliche Fermentation nach einem der Ansprüche 1 bis 12, wobei der Membrantrennschritt das Durchführen einer Filtrationsbehandlung durch Variieren des Drucks der Fermentationsflüssigkeit, die der Primärseite der Trennungsmembran zugeführt wird, umfasst.

**Revendications**

1. Procédé de production d'un produit chimique par fermentation continue, comprenant :

   une étape de fermentation pour convertir une charge de fermentation, par fermentation en mettant en culture un microorganisme ou des cellules de culture, en un liquide fermenté contenant le produit chimique par une cuve de fermentation ; et
   une étape de séparation membranaire pour collecter le produit chimique, sous forme de filtrat, à partir du liquide fermenté en utilisant une pluralité de modules à membranes de séparation, et pour renvoyer un liquide non filtré vers une cuve de fermentation ,
   dans lequel, dans l'étape de séparation membranaire, un traitement de filtration intermittente est réalisé de sorte qu'un traitement de filtration et un traitement d'arrêt de filtration soient répétés en alternance avec la pluralité de modules à membranes de séparation et en utilisant la pluralité de modules à membranes de séparation qui sont agencés en série, et
   le moment du traitement d'arrêt de filtration dans chaque module à membrane de séparation est contrôlé pendant le traitement de filtration intermittente.

2. Procédé de production d'un produit chimique par fermentation continue selon la revendication 1, dans lequel le traitement d'arrêt de filtration du module à membrane de séparation est contrôlé de sorte que l'arrêt de l'opération de filtration d'au moins un module à membrane de séparation soit réalisé pendant une opération de filtration d'un autre module à membrane de séparation.

3. Procédé de production d'un produit chimique par fermentation continue selon la revendication 1 ou 2, dans lequel le traitement d'arrêt de filtration du module à membrane de séparation est contrôlé de sorte que le traitement d'arrêt de filtration de chaque module à membrane de séparation ne se chevauche pas avec l'autre.

4. Procédé de production d'un produit chimique par fermentation continue selon la revendication 1, dans lequel, dans l'étape de séparation membranaire, le moment du traitement d'arrêt de filtration du module à membrane de séparation est contrôlé pour minimiser un changement de la quantité de liquide non filtré par heure, qui est renvoyée du module à membrane de séparation vers la cuve de fermentation pendant le traitement de filtration intermittente .

5. Procédé de production d'un produit chimique par fermentation continue selon l'une quelconque des revendications 1 à 4, dans lequel l'étape de séparation membranaire est réalisée par lavage à contre-courant en utilisant de l'eau comme liquide de lavage pendant le traitement d'arrêt de filtration.

6. Procédé de production d'un produit chimique par fermentation continue selon l'une quelconque des revendications 1 à 4, dans lequel l'étape de séparation membranaire est réalisée pendant le traitement d'arrêt de filtration par lavage à contre-courant en utilisant de l'eau contenant un agent oxydant ou un agent réducteur comme liquide de lavage.

7. Procédé de production d'un produit chimique par fermentation continue selon l'une quelconque des revendications 1 à 4, dans lequel l'étape de séparation membranaire est réalisée pendant le traitement d'arrêt de filtration par lavage à contre-courant en utilisant de l'eau contenant un acide ou un alcali comme liquide de lavage.

8. Procédé de production d'un produit chimique par fermentation continue selon l'une quelconque des revendications 1 à 4,

dans lequel l'étape de séparation membranaire est réalisée pendant le traitement d'arrêt de filtration par lavage par immersion en utilisant un liquide de lavage.

9. Procédé de production d'un produit chimique par fermentation continue selon l'une quelconque des revendications 5 à 7,
dans lequel, dans l'étape de séparation membranaire, le moment du traitement d'arrêt de filtration du module à membrane de séparation est contrôlé de manière à ce qu'une quantité de liquide non filtré qui est renvoyé du module à membrane de séparation vers la cuve de fermentation soit presque la même que la quantité de liquide de lavage qui est utilisé pour le lavage à contre-courant.

10. Procédé de production d'un produit chimique par fermentation continue selon la revendication 1,
dans lequel la pression transmembranaire est contrôlée de manière à être constante dans chacun des modules à membranes de séparation agencés en série.

11. Procédé de production d'un produit chimique par fermentation continue selon la revendication 1 ou 10,
dans lequel l'ordre de transport du liquide de fermentation vers une pluralité des modules à membranes de séparation agencés en série peut varier.

12. Procédé de production d'un produit chimique par fermentation continue selon l'une quelconque des revendications 1, 10 et 11,
dans lequel l'étape de séparation membranaire comprend la réalisation, sur plusieurs lignes en parallèle, d'une opération de filtration intermittente en utilisant une unité de membrane de séparation qui comporte une pluralité de modules à membranes de séparation qui sont agencés en série.

13. Procédé de production d'un produit chimique par fermentation continue selon l'une quelconque des revendications 1 à 12,
dans lequel l'étape de séparation membranaire comprend la réalisation d'un traitement de filtration en faisant varier la pression du liquide de fermentation fourni à un côté principal de la membrane de séparation.

FIG.1

# FIG.2

```
                    START

FILTRATION TREATMENT WITH EVERY          S1
SEPARATION MEMBRANE MODULE

FILTRATION-STOP TREATMENT WITH           S2
SEPARATION MEMBRANE MODULE 2A

FILTRATION TREATMENT WITH EVERY          S3
SEPARATION MEMBRANE MODULE

FILTRATION-STOP TREATMENT WITH           S4
SEPARATION MEMBRANE MODULE 2B

FILTRATION TREATMENT WITH EVERY          S5
SEPARATION MEMBRANE MODULE

FILTRATION-STOP TREATMENT WITH           S6
SEPARATION MEMBRANE MODULE 2C

                     END
```

# FIG.3

START

↓

FILTRATION TREATMENT WITH EVERY
SEPARATION MEMBRANE MODULE — S11

↓

BACK PRESSURE WASHING WITH
SEPARATION MEMBRANE MODULE 2A — S12

↓

FILTRATION TREATMENT WITH EVERY
SEPARATION MEMBRANE MODULE — S13

↓

BACK PRESSURE WASHING WITH
SEPARATION MEMBRANE MODULE 2B — S14

↓

FILTRATION TREATMENT WITH EVERY
SEPARATION MEMBRANE MODULE — S15

↓

BACK PRESSURE WASHING WITH
SEPARATION MEMBRANE MODULE 2C — S16

↓

END

FIG.4

FIG.5

EP 2 657 343 B1

FIG.6

EP 2 657 343 B1

# FIG.7

```
            ┌─────────────┐
            │    START    │
            └─────────────┘
                   │
                   ▼
    ┌──────────────────────────────────┐
    │  FILTRATION TREATMENT WITH EVERY  │ ⌐S21
    │     SEPARATION MEMBRANE MODULE    │
    └──────────────────────────────────┘
                   │
                   ▼
    ┌──────────────────────────────────┐
    │   FILTRATION-STOP TREATMENT WITH  │
    │ SEPARATION MEMBRANE MODULES 2A    │ ⌐S22
    │              AND 2D               │
    └──────────────────────────────────┘
                   │
                   ▼
    ┌──────────────────────────────────┐
    │  FILTRATION TREATMENT WITH EVERY  │ ⌐S23
    │     SEPARATION MEMBRANE MODULE    │
    └──────────────────────────────────┘
                   │
                   ▼
    ┌──────────────────────────────────┐
    │   FILTRATION-STOP TREATMENT WITH  │
    │ SEPARATION MEMBRANE MODULES 2B    │ ⌐S24
    │              AND 2E               │
    └──────────────────────────────────┘
                   │
                   ▼
    ┌──────────────────────────────────┐
    │  FILTRATION TREATMENT WITH EVERY  │ ⌐S25
    │     SEPARATION MEMBRANE MODULE    │
    └──────────────────────────────────┘
                   │
                   ▼
    ┌──────────────────────────────────┐
    │   FILTRATION-STOP TREATMENT WITH  │
    │ SEPARATION MEMBRANE MODULES 2C    │ ⌐S26
    │              AND 2F               │
    └──────────────────────────────────┘
                   │
                   ▼
            ┌─────────────┐
            │     END     │
            └─────────────┘
```

# FIG.8

```
            ┌─────────────┐
            │    START    │
            └──────┬──────┘
                   ▼
  ┌──────────────────────────────────┐
  │  FILTRATION TREATMENT WITH EVERY  │  ～S31
  │    SEPARATION MEMBRANE MODULE     │
  └──────────────┬───────────────────┘
                 ▼
  ┌──────────────────────────────────┐
  │     BACK PRESSURE WASHING WITH     │
  │ SEPARATION MEMBRANE MODULES 2A    │  ～S32
  │             AND 2D                 │
  └──────────────┬───────────────────┘
                 ▼
  ┌──────────────────────────────────┐
  │  FILTRATION TREATMENT WITH EVERY  │  ～S33
  │    SEPARATION MEMBRANE MODULE     │
  └──────────────┬───────────────────┘
                 ▼
  ┌──────────────────────────────────┐
  │     BACK PRESSURE WASHING WITH     │
  │ SEPARATION MEMBRANE MODULES 2B    │  ～S34
  │             AND 2E                 │
  └──────────────┬───────────────────┘
                 ▼
  ┌──────────────────────────────────┐
  │  FILTRATION TREATMENT WITH EVERY  │  ～S35
  │    SEPARATION MEMBRANE MODULE     │
  └──────────────┬───────────────────┘
                 ▼
  ┌──────────────────────────────────┐
  │     BACK PRESSURE WASHING WITH     │
  │ SEPARATION MEMBRANE MODULES 2C    │  ～S36
  │             AND 2F                 │
  └──────────────┬───────────────────┘
                 ▼
            ┌─────────────┐
            │     END     │
            └─────────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007252367 A
- JP 2008237101 A
- JP 2009065966 A
- JP 2008161071 A
- JP 2009072708 A

**Non-patent literature cited in the description**

- **TOSHIHIKO HIRAO et al.** *Appl. Microbiol. Biotechnol.,* 1989, vol. 32, 269-273